# EUROPEAN PATENT APPLICATION

(11) **EP 4 344 535 A1**
(43) Date of publication of application: **03.04.2024**
(21) Application number: 22198172.3
(22) Date of filing: 27.09.2022
(51) Int. Cl.: A01G 18/00, A61K 36/07, C12N 1/14

(54) **METHODS AND SUBSTRATES FOR INDUCING AND GROWING FRUITING BODIES OF THE FUNGUS LAETIPORUS SULPHUREUS AS VEGAN MEAT SUBSTITUTE**

(71) Applicant: Walding Foods GmbH, 85354 Freising (DE)
(72) Inventor: STILLE, Alison, 85354 Freising (DE); STILLE, David, 85354 Freising (DE); AMAN, Johannes, 85354 Freising (DE)
(74) Representative: Vossius & Partner Patentanwälte Rechtsanwälte mbB

(57) **Abstract**

The present invention relates to methods and/or substrates for inducing and growing fungi of the genus *Laetiporus*, including *Laetiporus sulphureus.* Fruiting in the methods of the present invention is induced by a decrease in the CO₂ concentration during indoor/in vitro mushroom culture. The present invention further relates to culture substrates comprising a cellulose component, an amino acid component, a mineral component and, optionally, a fibrous component. The present invention further relates to fruiting bodies produced by performing the methods and/or using the substrates of the present invention, i.e., a fruiting body obtained by or obtainable by the method provided herein. The present invention is particularly useful for the industrial production of mushrooms of the genus *Laetiporus*, including *Laetiporus sulphureus* in food-grade quality, and as such as foodstuff.

## Description

### Field of Invention

The present invention relates to the reproducible large-scale indoor production of fruiting bodies of the fungus *Laetiporus sulphureus* or other fungi of the genus *Laetiporus* as foodstuff.

### Background

With an increased awareness of the damaging impacts of the current animal food product industries on health and the environment at large, more and more consumers are opting for plant-based food options for both, health and ethical reasons. Various food companies are trying to respond to this demand by producing cell- and/or fermentation-based meat substitutes and/or plant-based meat alternatives, such as tofu or seitan. Currently available meat substitutes are mostly based on highly processed products including artificial additives to enhance flavor, color, and texture. Hence, there is a great need for new, sustainable, healthy and tasty meat alternatives.

### Laetiporus sulphureus

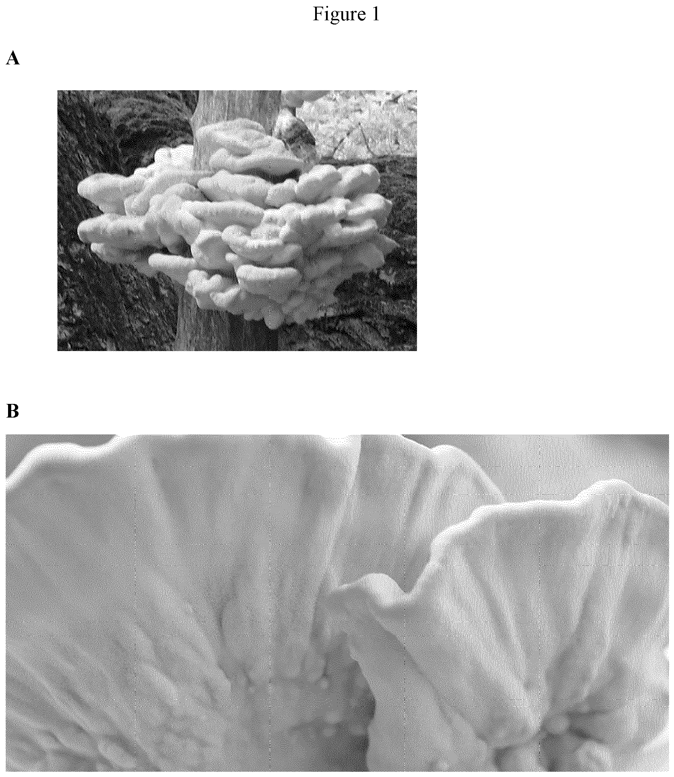

The fungus *Laetiporus sulphureus* is a member of the class Agaricomycetes of the division Basidiomycota, group/order *Polyporales* and genus *Laetiporus.* Wild *Laetiporus sulphureus* typically grows seasonally between May and October on deciduous and coniferous trees, as well as on dead logs (NPL8, NPL13) (see **Figure 1A**). Wild *Laetiporus sulphureus* is commonly found throughout North America, Europe, and Asia.

*Laetiporus sulphureus* was traditionally used as therapeutic agent against pyretic diseases, coughs, gastric cancer, and rheumatism (NPL1, NPL2). Furthermore, the fruiting bodies of *Laetiporus sulphureus* were discovered to exhibit medicinal properties including, inter alia, immunomodulation, antitumor, hemagglutination, anticoagulation, antioxidant, antibacterial, antifungal, insecticidal, anti-ulcer, insulin tropic anti-HIV, and cytostatic activities (NPL1, NPL3, NPL4, NPL5, NPL3).

The fruiting bodies of *Laetiporus sulphureus* are typically fleshy, sulfuric-yellow, semi-circular hats, that eventually form shelf-shaped, bracket-like bodies (NPL1), which typically have no gills, but rather tiny pores (NPL7) (see **Figure 1B**). In contrast to most members of the fungal group *Polypores* which are inedible due to their hard and corky texture, young fruiting bodies of *Laetiporus sulphureus* are widely praised for their high nutritional value as well as their chicken-like texture and taste (NPL3, NPL8, NPL9, NPL10). Due to the latter characteristic, *Laetiporus sulphureus* is commonly referred to as "Chicken-of-the-Woods" and as such has a great value as vegan/vegetarian alternative for meat, since no artificial additives to enhance taste and/or texture are required (NPL11). Remarkably, young fruiting bodies of *Laetiporus sulphureus* provide key nutritional components such as carbohydrates, proteins including essential amino acids, vitamins, minerals, essential fatty acids and dietary fiber (NPL12).

Although a praised and highly sought-after mushroom, there is very little literature on the cultivation, particularly indoor/in vitro cultivation, of *Laetiporus sulphureus* and its fruiting bodies are predominantly available as specimen collected from the wild.

Two publications mention the formation of fruiting bodies of *Laetiporus sulphureus* on wood after long-term outdoor cultivation in an experimental setup (NPL7, NPL14). Outdoor cultivation, however, is not suitable for a large-scale production of food-grade fruiting bodies, because the incubation period until the first formation of fruiting bodies may take up to several years, and the quality as well as the yield drastically vary depending on the prevailing environmental conditions.

With respect to indoor cultivation methods, it is reported that *Laetiporus sulphureus* fruiting bodies spontaneously formed on a substrate based on sunflower shells (NPL15). However, further details on the composition of the substrate and growing conditions seem to be missing from this study.

Furthermore, Chinese patents CN 102391049 and CN 102405766 are directed to the in vitro production of fruiting bodies of *Laetiporus sulphureus* based on, inter alia, wood-based substrates. In addition, CN 102391049 discloses the use of Zinc sulfate (ZnSO₄) as mineral component in the substrate, which is known to cause acute stomach distress including nausea and vomiting when being consumed in too large amounts. Thus, production of fruiting bodies suitable for human consumption based on the substrate disclosed in CN 102391049 is questionable. The substrate disclosed in CN 102405766 does not seem to contain mineral components at all. However, mineral components act as buffer to counteract substrate acidification arising due to mycelial growth. In view of the absence of mineral components in the substrate, the reported increase of the pH after the mycelial growth phase reported in CN 102405766 remains debatable. Additionally, in order to induce fruiting body formation, the cultivation bags in CN 102405766 are cut open. However, since *Laetiporus sulphureus* is easily contaminated by other fungal species, any opening of the cultivation vessels during cultivation ultimately results in contaminations and spoilage of the emerging fruiting bodies (NPL16).

The Polish patent PL 219753 relates to a previously published report (NPL16) and is based on a substrate containing, inter alia, wooden components from oak, i.e., oak sawdust, which according to common general knowledge is rich in tannins. Emerging fruiting bodies adsorb these tannins from the culture substrate and consequently, may be inedible due to their bitter taste. Furthermore, the substrate disclosed in PL 219753 contains a variety of different ingredients not all of which are readily available on a bigger scale, such as buckwheat bran and the triticale berry - a hybrid of wheat and rye - and wood dust of several different tree species. Moreover, formation of fruiting bodies from *Laetiporus sulphureus* mycelium in PL 219753 is said to be either induced by a decrease in temperature from 2 to 4 °C for 24 h or by cold water injection into the cultivation vessel. Compared to fruiting body induction via temperature decrease, the injection of cold water is reported to result in an increased yield. Yet, injecting water is a labor-intensive step that additionally involves a high risk of contamination impacting the reproducibility of the method disclosed in PL 219753.

Thus, there is a need for improved or alternative methods and/or improved or alternative substrates for reproducible, robust and scalable induction of fruiting bodies of fungi of the genus *Laetiporus,* including *Laetiporus sulphureus,* and subsequent production of this fungal species in food-grade quality. Accordingly, the technical problem underlying the present invention is the provision of improved means and methods for the induction of the formation and growth of fruiting bodies of fungi of the genus *Laetiporus,* including *Laetiporus sulphureus.*

The technical problem is solved by the provision of the embodiments characterized in the claims and the embodiments described hereinbelow.

### Summary of the Invention

The present invention relates to methods and/or substrates for inducing and growing fungi of the genus *Laetiporus,* including *Laetiporus sulphureus.* Fruiting in the methods of the present invention is induced by a decrease in the CO₂ concentration during indoor/in vitro mushroom culture. The present invention further relates to culture substrates comprising a cellulose component, an amino acid component, a mineral component and, optionally, a fibrous component. The present invention further relates to fruiting bodies produced by performing the methods and/or using the substrates of the present invention, i.e., a fruiting body obtained by or obtainable by the method provided herein. The present invention is particularly useful for the industrial production of mushrooms of the genus *Laetiporus,* including *Laetiporus sulphureus,* in food-grade quality, and as such as foodstuff.

Provided herein are new methods and substrates for the industrial production of fruiting bodies of fungi of the genus *Laetiporus* and fungi of the species *Laetiporus sulphureus* as well as fruiting bodies obtainable by using said methods and substrates. Further preferred embodiments of the present invention are defined in dependent claims and items. In particular, the present invention solves the technical problem of economically producing fruiting bodies of the fungus *Laetiporus sulphureus* in large scale and food-grade quality.

In particular, the methods/substrates of the present invention provide improved induction and subsequent growth of fruiting bodies, preferably from *Laetiporus sulphureus* during indoor cultivation which could not have been achieved by previously known methods and/or substrates. Further, the fruiting bodies obtained or obtainable by the present invention are rich in protein and safe for human consumption, since the substrates preferably to be used for cultivation do not contain ingredients of potential concern, such as toxic wood components and toxic mineral components and/or components which would render the fruiting body unsuitable for human consumption.

The present invention can be described/summarized by the following items:
[1] A method for inducing fruiting body formation of a fungus from the genus *Laetiporus* comprising the steps:
   a) incubating a substrate inoculated with the fungus at a first predetermined CO₂ concentration; and
   b) incubating the substrate at a second predetermined CO₂ concentration,
   wherein the second predetermined CO₂ concentration is lower than the first predetermined CO₂ concentration.
[2] The method according to item [1], wherein the first predetermined CO₂ concentration in step a) is above atmospheric levels, preferably above 700 ppm, more preferably above 1000 ppm, even more preferably about 1500 ppm, or above 2500 ppm and up to 5000 ppm, or up to 10000 ppm.
[3] The method according to item [1] or [2], wherein the second predetermined CO₂ concentration in step b) is below 1500 ppm, preferably below 1000 ppm, more preferably below 700 ppm, more preferably at atmospheric levels.
[4] The method according to any one of the preceding items, wherein the method comprises a step of decreasing the first predetermined CO₂ concentration to a second predetermined CO₂ concentration.
[5] The method according to item [4], wherein the CO₂ concentration is decreased within 5 days, preferably within 24 hours, preferably within 12 hours, even more preferably within 3 hours.
[6] The method according to any one of the preceding items, wherein the incubation in step a) is performed at a first predetermined temperature and/or a first predetermined humidity.
[7] The method according to item [6], wherein the first predetermined temperature is 18 °C to 30 °C, preferably 22 °C to 28 °C, more preferably is 26 °C and/or the first predetermined humidity is 35 to 80 %, preferably 40 to 70 %, more preferably 45 to 55 %, most preferably at 50%.
[8] The method according to any one of the preceding items, wherein the incubation in step a) is performed in the dark or under low light conditions, preferably below 100 lux.
[9] The method according to any one of the preceding items, wherein the incubation in step a) is performed at least 2 weeks, preferably at least 2 to 3 weeks, more preferably 20 days.
[10] The method according to any one of the preceding items, wherein the incubation in step b) is performed at a second predetermined temperature and/or a second predetermined humidity and/or under high light conditions, preferably above 100 lux.
[11] The method according to item [10], wherein the second predetermined temperature is lower than the first predetermined temperature and/or the second predetermined humidity is higher than the first predetermined humidity and/or high light conditions are higher than the dark or low light conditions used in step (a).
[12] The method according to item [11], wherein the second predetermined temperature is 12 °C to 26 °C, preferably 16 °C to 22 °C, more preferably is 18 °C and/or the second predetermined humidity is above 40 %, preferably above 50 %, more preferably above 70 %, even more preferably above 80 %, most preferably is 90% and/or wherein the high light conditions is preferably above 100 lux, more preferably above 500 lux and preferably up to 3000 lux for 10 to 14 hours per day.
[13] The method according to any one of the preceding items, wherein the substrate has been sterilized prior to inoculation.
[14] The method according to any one of the preceding items, wherein the substrate is provided in a sterilizable vessel.
[15] The method according to item [14], wherein the sterilizable vessel is selected from the group consisting of polypropylene bags and polymer containers.
[16] The method according to item [15], wherein the sterilizable vessel is a polypropylene bag, preferably further comprising one or more filter patches, preferably with a mesh size of 2 µm to 50 µm.
[17] A method for growing a fruiting body of a fungus of the genus *Laetiporus* comprising the steps:
   i) inducing fruiting body formation according to the methods for inducing fruiting body formation of the present invention, preferably according to items 1 to 16;
   ii) incubating the substrate until a fruiting body has grown;
   iii) harvesting the fruiting body.
[18] The method according to item [17], wherein the incubation in step ii) is performed at the second predetermined CO2 concentration, a second predetermined temperature and/or a second predetermined humidity.
[19] The method according to item [18], wherein the second predetermined temperature is 12 °C to 26 °C, preferably 16 °C to 22 °C, more preferably is 18 °C and/or the second predetermined humidity is above 40 %, preferably above 50 %, more preferably above 70 %, even more preferably above 80 %, most preferably is 90%.
[20] The method according to any one of items [17] to [19], the incubation in step ii) is performed at 0 to 3000 lux, preferably at 500 to 3000 lux for 10 to 14 hours per day.
[21] The method according to any one of the preceding items, wherein the substrate comprises:
   a) a cellulose component;
   b) an amino acid component; and
   c) a mineral component;
   d) and, optionally, a fibrous component.
[22] A fruiting body obtained by or obtainable by the method according to any one of items [1] to [21].
[23] A fruiting body of a fungus from the genus *Laetiporus,* the fruiting body comprising at least 5 %, preferably at least 7 %, more preferably at least 10 %, even more preferably at least 12 % of protein per gram of wet sample and/or
   comprising above 21 %, more preferably above 25 % of protein per gram of dry weight.
[24] The method according to any one of items [1] to [21], or the fruiting body according to item [22] or [23], wherein the fungus is from the species *Laetiporus sulphureus.*
[25] A substrate comprising:
   a) a cellulose component;
   b) an amino acid component; and
   c) a mineral component;
   d) and, optionally, a fibrous component.
[26] The substrate according to item [25], wherein the cellulose component is selected from the group consisting of wood chips, saw dust and/or combinations thereof, preferably from trees selected from the group consisting of alder, ash, beech, birch, fruit trees (e.g., apple, pear, plum), poplar, willow, fir, larch, pine, and/or combinations thereof, preferably wherein the cellulose component is beech.
[27] The substrate according to item [25] or [26], wherein the cellulose component is not derived from timbers containing toxins such as yew, robinia and the like and/or containing a high amount of tannic acid such as oak, chestnut and the like.
[28] The substrate according to item [26], wherein the size of the wood chips is preferably between 0.2 and 30 mm.
[29] The substrate according to any one of the preceding items, wherein the amount of the cellulose component is between 25 % to 75 % of the total dry weight of the substrate, preferably between 35 to 65 % of the total dry weight of the substrate, more preferably between 45 to 55 % of the total dry weight of the substrate.
[30] The substrate according to any one of the preceding items, wherein the amino acid component is be selected from the group consisting of grains, bran, malt, meal, algae, dried herbal matter (e.g., alfalfa hay), spirulina powder, nutritional yeast (e.g., beer yeast), nuts, beans, legumes and/or combinations thereof.
[31] The substrate according to item [30], wherein the grains are selected from the group consisting of wheat, rye, rice, millet, barley, oats, corn, buckwheat, quinoa, chia, and/or combinations thereof.
[32] The substrate according to item [30], meal is selected from the group consisting of lin, pumpkin, coco, poppy, rape, sunflower, sesame, thistle, soy, palm fruits, hemp seeds and/or combinations thereof.
[33] The substrate according to any one of the preceding items, wherein the amount of the amino acid component is between 1 % to 50 % of the total dry weight of the substrate, preferably between 8 % to 47.5 % of the total dry weight of the substrate.
[34] The substrate according to any one of the preceding items, wherein the mineral component is selected from the group consisting of chalk, calcium carbonate (CaCO₃), dolomite, gypsum, ground seashells, coralligene, bone and/or other natural sources of calcium carbonate, magnesium carbonate, calcium sulphate and potassium sulphate and/or combinations thereof.
[35] The substrate according to any one of the preceding items, wherein the amount of the mineral component is between 1 % to 10 % of the total dry weight of the substrate, preferably between 2.5 % to 7.5 % of the total dry weight of the substrate, more preferably between 4.5 to 5.5 % of the total dry weight of the substrate.
[36] The substrate according to any one of the preceding items, wherein the fibrous component is selected from the group consisting of hemp, wood fiber, peat, paper or cardboard, cotton hulls, jute, banana, coco, bagasse, flax, reed, nettle, straw, hay and/or combinations thereof.
[37] The substrate according to any one of the preceding items, wherein the amount of the fibrous component is between 2 % to 45 % of the total dry weight of the substrate, preferably between 4 % to 30 % of the total dry weight of the substrate, more preferably between 5 to 25 % of the total dry weight of the substrate.
[38] The substrate according to any one of the preceding items, wherein the substrate further comprises a predetermined water content.
[39] The substrate according to item [38], wherein the predetermined water content is between 30 % to 70 %, preferably between 35 % to 55 %, more preferably 40 % to 50 %, even more preferably 40 % to 45 %, most preferably 43 %.
[40] The substrate according to any one of the preceding items, wherein the substrate further comprises a saccharated component, preferably selected from the group consisting of glucose, fructose, galactose, dextrose, maltose, sucrose, lactose, polyalcohols, glycosides, N-acetylglucosamine and/or combinations thereof.
[41] The substrate according to item [40], wherein the amount of the saccharated component is between 0.25 % to 5 % of the total dry weight of the substrate, preferably between 0.5 % to 2 % of the total dry weight of the substrate, more preferably between 0.8 to 1.5 % of the total dry weight of the substrate.

Some particular aspects of the invention can be summarized as follows:
In a first aspect the present invention relates to a method for inducing fruiting body formation of a fungus from the genus *Laetiporus.* The method of the first aspect comprises the following steps:
a) incubating a substrate inoculated with the fungus at a first predetermined CO₂ concentration; and
b) incubating the substrate at a second predetermined CO₂ concentration,
wherein the second predetermined CO₂ concentration is lower than the first predetermined CO₂ concentration.

In some aspects of the present invention, the first predetermined CO₂ concentration in step a) is above atmospheric levels, preferably above 700 ppm, more preferably above 1000 ppm, even more preferably above 1500 ppm, above 2500 ppm and up to 5000 ppm or 10000 ppm.

In some aspects of the present invention, the second predetermined CO₂ concentration in step b) is below 1500 ppm, preferably below 1000 ppm, more preferably below 700 ppm, more preferably at atmospheric levels.

In some aspects of the present invention, the method of the first aspect of the present invention comprises a step of decreasing the first predetermined CO₂ concentration to a second predetermined CO₂ concentration.

In some aspects of the present invention, the CO₂ concentration is decreased within 5 days, preferably within 24 hours, preferably within 12 hours, even more preferably within 3 hours.

In a second aspect, the present invention relates to a method for growing the induced fruiting bodies of the fungus.

The method of the second aspect of the present invention comprises the following steps:
i) inducing fruiting body formation according to the methods for inducing fruiting body formation of the present invention;
ii) incubating the substrate until a fruiting body has grown;
iii) harvesting the fruiting body.

In some aspects of the present invention, the incubation in step ii) is performed at the second predetermined CO2 concentration, a second predetermined temperature and/or a second predetermined humidity

In some aspects of the present invention, the second predetermined temperature is 12 °C to 26 °C, preferably 16 °C to 22 °C, more preferably is 18 °C and/or the second predetermined humidity is above 40 %, preferably above 50 %, more preferably above 70%, even more preferably above 80 %, preferably is 90 %.

In a third aspect, the present invention relates to substrates for inducing and growing a fruiting body of a fungus.

The substrate according to the present invention comprises the following components:
a) a cellulose component;
b) an amino acid component; and
c) a mineral component;
d) and, optionally, a fibrous component.

In some aspects of the present invention, the substrates according to the present invention further comprises a predetermined water content.

In some aspects of the present invention, the predetermined water content is between 30 % to 70 %, preferably between 35 % to 55 %, more preferably 40 % to 50 %, even more preferably 40 % to 45 %, most preferably 43 %.

In a fourth aspect, the present invention relates to a fruiting body obtained by or obtainable by performing the methods, i.e., the induction and/or growth methods, disclosed herein.

In a fifth aspect, the present invention relates to a fruiting body of a fungus from the genus *Laetiporus.*

In some aspects of the present invention, the fruiting body comprises at least 5 %, preferably at least 7 %, more preferably at least 10 %, even more preferably at least 12 % of protein per gram of wet sample/fresh weight and preferably up to 10, 11, 12, 13, 14 or 15 % of protein per gram of wet sample/fresh weight.

Additionally, and/or alternatively a fruiting body of a fungus comprises above 21 %, more preferably above 25 %, 30 % 35 %, 40 %, 45 %, 50 % of protein per gram of dry weight and up to 55 % or 60 % protein per gram of dry weight.

In the context of the present invention the fungus is from the genus *Laetiporus,* more preferably from the species *Laetiporus sulphureus.* Likewise, the fruiting body provided herein or obtained/obtainable by the herein provided methods is from a fungus from the genus *Laetiporus,* more preferably from the species *Laetiporus sulphureus.*

### Brief description of the Figures

In the following, preferred embodiments of the present invention are described in detail with respect to the figures.
Figure 1 Fruiting bodies of the fungus *Laetiporus sulphureus.* (A) Cluster of wild growing mature shelf-shaped fruiting bodies of the tree fungus *Laetiporus sulphureus* on a dead log. (B) Two *Laetiporus sulphureus* fruiting bodies with sulfuric-yellow, semi-circular hats and tube-like pores at the undersurface.

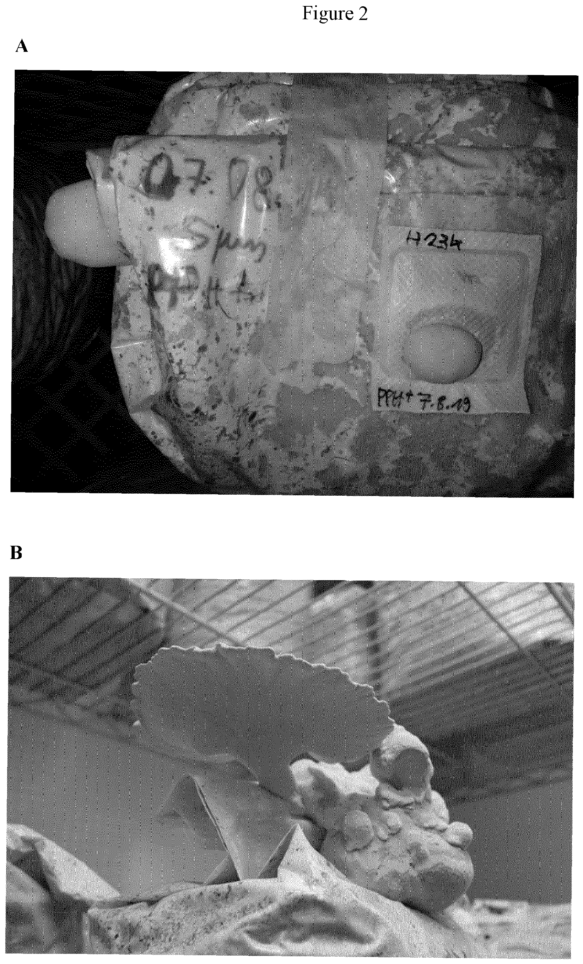

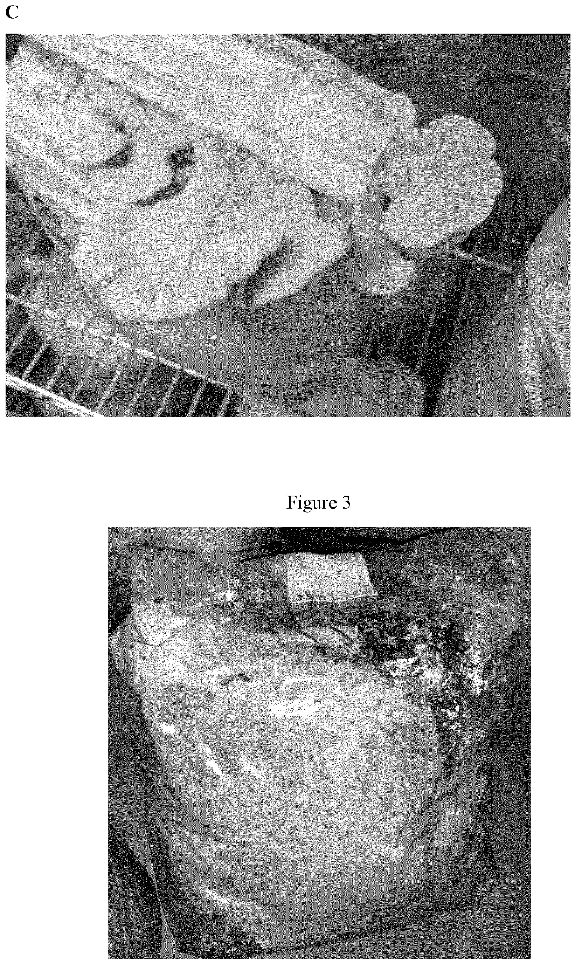

Figure 2 **In vitro induced fruiting bodies of *Laetiporus sulphureus* according to the present invention. (A)** Primordia of the fungal species *Laetiporus sulphureus* growing out of filter patches and folds of the cultivation vessel. Fruiting bodies of the fungal species *Laetiporus sulphureus* growing out of **(B)** filter patches and **(C)** folds of the cultivation vessel.
Figure 3 **In vitro induced fruiting bodies of *Laetiporus sulphureus* according to a method and a substrate known in the art** (PL 219753). Culture vessel in which fruiting of the fungal species *Laetiporus sulphureus* was induced by using a method and a substrate known in the art: Contaminations (black parts/structures/matter) overgrowing *Laetiporus* mycelium after fruiting body induction via cold water injection.

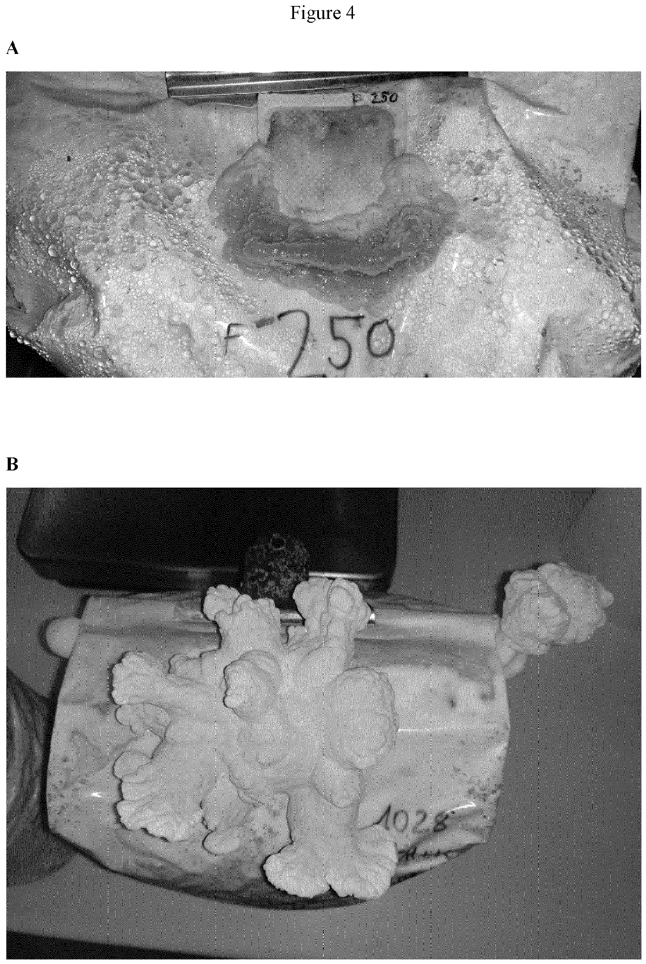

Figure 4 **In vitro induced fruiting bodies of *Laetiporus sulphureus* according to the present invention based on different substrates.** Fruiting bodies induced and grown based on (**A**) a substrate known in the art (PL 219753) and (**B**) substrates provided herein.

### Detailed description of the Invention

The present invention provides methods and substrates for inducing and subsequently growing fruiting bodies of a fungus, preferably *Laetiporus sulphureus.* In particular, the methods and substrates of the present invention are useful for the in vitro/indoor production of *Laetiporus sulphureus* fruiting bodies in food-grade quality, which was not achieved by previously known methods and substrates. The methods of the present invention enable to reproducibly induce and grow fruiting bodies of the fungus *Laetiporus sulphureus* with 20 to 50 % reduced production time compared to culture methods known in the art. Furthermore, the fruiting bodies of the fungus *Laetiporus sulphureus* produced by using the substrates of the present invention are safe for human consumption and rich in protein.

### Fungus

In the context of the present invention the terms "fungus (fungi)" and "mushroom(s)" are used interchangeably.

Mushrooms grow from spores, which sprout and grow thin whitish fibers called hyphae. Hyphae of compatible mating types join and branch into a network of thin threads called mycelium. In other words, the mycelium has a root-like structure and consists of a mass of branching, thread-like hyphae. Thus, the mycelium is the growing part of a mushroom by which the fungus absorbs nutrients from its surrounding environment. Hyphae further forming into knots, also referred to as primordia, finally develop to the fruiting body of the mushroom. Fruiting bodies are the multicellular structure on which spore-producing structures (in the case of Basidiomycetes, basidia are borne) and as such are the reproductive part of a fungus. The spores dispersing from the mature fruiting bodies initiate the aforementioned life cycle again. Besides being the reproductive part of the fungus, the fleshy fruiting bodies are usually the consumed part of edible fungal species, although developing, unmature fruiting bodies and primordia may also be consumed.

Fungi according to the present invention can be selected from the subkingdom *Dikarya,* the division *Basidiomycota,* the class *Agaricomycetes,* the order/group *Polyporales,* the family *Fomitopsidaceae,* the genus *Laetiporus* and the species *Laetiporus sulphureus.*

In a preferred embodiment of the present invention, in vitro cultured fruiting bodies are preferably selected from the genus *Laetiporus,* more preferably from the species *Laetiporus sulphureus.*

The skilled person will understand that each mushroom species may have different growth requirements. Therefore, the culture conditions need to be determined/adapted depending on the destined species to be cultivated.

### Mushroom cultivation

Cultivation of edible mushroom species commonly comprises several particular steps. First, mycelium of the desired fungal species is cultivated on a carrier medium, such as grains, in order to produce spawn. Next, a different, usually sterile substrate, is inoculated with the produced spawn. The inoculated mushroom mycelium then colonizes the substrate from the spawn propagules. When the substrate is fully colonized by the mycelium, the formation of primordia is induced via a change of the culture conditions. Most commonly in the art, induction of mushroom fruiting is initiated by a combination of a decrease in temperature and an increase in humidity. After formation of primordia, to balance the growth of the fruiting bodies the temperature may be increased again.

In the context of the present invention, cultivation of mushrooms relates to the indoor/in vitro cultivation of mushrooms, i.e., the cultivation in closed environments in which culture conditions (e.g., ventilation, temperature, relative humidity and light) can be tightly controlled and regulated (e.g., via sensors). Closed environments may include, but are not limited to, incubation chambers, greenhouses, incubation rooms and the like. According to the present invention, incubation rooms/incubators, which may have, for instance, a size of 14 m², are particularly preferred.

The mushrooms in the context of the present invention have an aerobic metabolism, meaning that oxygen is metabolized to, inter alia, carbon dioxide (CO₂). In order to prevent malformation and atrophy of the fruiting bodies, oxygen supply and CO₂ removal must be tightly regulated. This may be done via ventilation systems, such as industrial fans in combination with precise CO₂ measurements, such as CO₂ sensors, which may be placed at the cultivation vessels, and/or optionally, which may be placed in the cultivation vessels.

The skilled person will understand that the CO₂ concentration within the culture environment may be set adjusted based on ventilation. More specifically, if no/moderate ventilation is performed herein, the CO₂ concentration within the culture environment increases due to the mushrooms' metabolism. Whereas, when ventilation is increased the CO₂ levels can be lowered. In this context, ventilation means in particular that (filtered) atmospheric air (e.g., air with a lower CO₂ level compared to the air in the culture environments (e.g., incubation room)) is allowed to enter the culture environment (e.g., incubation room/incubator).

The culture environments provided herein may be humidified and any means suitable for humidifying known in the art may be employed. For instance, the humidity in the culture environment of the present invention may be adjusted via humidifiers, including but not limited to, evaporative humidifiers, impeller humidifiers, ultrasonic humidifiers, high pressure mist producing systems and the like. According to the present invention, mist producing systems are preferred.

The culture environment in the context of the present invention may be illuminated and any means suitable for illumination known in the art, such as a natural or an artificial light source, may be employed. According to the present invention an artificial dimmable light source with a broad spectrum is particularly useful.

The skilled person is in the position to derive mycelium from a desired fungal species from which spawn is to be produced. In the context of the present invention, mycelium of the desired fungal species may be isolated from wild picked mushrooms, e.g., via incubation of spores or fungal matter on sterile medium in vessels/petri dishes or may be obtained from any other source known in the art.

Means for producing spawn, i.e., the mycelial matter to be vaccinated on the culture substrate also referred to as inoculate, is not limited and any suitable means known to the skilled person, may be employed. In the context of the present invention, the produced spawn may include, but is not limited to, sawdust spawn, grain spawn, stick spawn, liquid spawn and the like. According to the present invention, grain spawn is particularly useful.

### Induction Method / Fruiting bodies

In a first aspect, the present invention relates to a method for inducing fruiting body formation of a fungus which is triggered/induced by a decrease in the CO₂ concentration during the culture process.

The method of the first aspect of the present invention comprises the following steps:
a) incubating a substrate inoculated with the fungus at a first predetermined CO₂ concentration; and
b) incubating the substrate at a second predetermined CO₂ concentration,
wherein the second predetermined CO₂ concentration is lower than the first predetermined CO₂ concentration.

In the context of the present invention, the terms, "inducing fruiting body formation" and "fruiting" are used interchangeably and refer to the development/formation/appearance of primordia, i.e., hyphal knots marking the initial, immature stage of the mushroom's fruiting bodies which typically have the size of a pea (e.g., diameter of about 4.0 to 7.5 mm).

In the context of the present invention, "fruiting body" preferably refers to the mature/fully differentiated stage of mushroom. The skilled person will understand that in the context of the present invention, fruiting bodies are usually the fleshy consumed/consumable part of edible fungal species; the fruiting bodies typically consist of a dense/compact network of the mycelial matter of the fungus and/or are the mature/fully differentiated stage of mushroom and/or are larger in size compared to the above-mentioned primordia (i.e. compared to the initial stage of fruiting body formation). Also developing, immature fruiting bodies may be consumed, as it is custom with other fungi species. Accordingly, in the context of the present invention, "fruiting body" can also refer to developing and/or immature fruiting bodies. "Developing and/or immature fruiting body" in this context refers primarily to amorphous, developing and/or immature fruiting body (also termed herein amorphous fruiting body or amorphous immature fruiting body or amorphous developing fruiting body), e.g., fruiting bodies grown e.g., in the dark or under low light conditions (preferably < 100 lux), e.g., when performing step (ii) of the methods provided herein. "Developing and/or immature fruiting body" are usually larger in size compared to the primordia which are the initial, immature stage of the mushroom's fruiting bodies, as explained above. For example, a "developing and/or immature fruiting body" can have a diameter of above 7.5 mm and up to several cm, e.g., up to 10, 15 or 20 cm). Without deferring from the gist of invention, it is contemplated that (a sample of) "fruiting bodies" (e.g., to be harvested in accordance with the provided methods) as defined herein can, in addition, comprise primordia (in the sense of initial, immature stage of the mushroom's fruiting bodies or parts thereof). Preferably, (a sample of) "fruiting bodies" (e.g., to be harvested in accordance with the provided methods) as defined herein does not comprise primordia (in the sense of initial, immature stage of the mushroom's fruiting bodies or parts thereof).

A "fruiting body" herein preferably is a fresh weight fruiting body, i.e., a freshly harvested fruiting body (e.g., from shortly after harvest (like several minutes) to up to several weeks, particularly when stored at low temperatures (e.g., at 4 °C)), as compared to a dry/dried fruiting body. Most preferably, the "fruiting body" is pasteurized (directly) after harvest. Such a pasteurized fruiting body can then be stored for up to several weeks, particularly when stored at low temperatures (e.g., at 4 °C).

Fruiting body induction can be visually observed/determined e.g., by emergence of primordia (i.e., initial stage of fruiting bodies).

The percentage of induction efficiency can be calculated/determined based on the ratio of samples (e.g., inoculated culture vessels (e.g., polypropylene bags) with emerging fruiting bodies (e.g., appearance/development of primordia) and the total sample size (e.g., total number of inoculated culture vessels (e.g., polypropylene bags). Induction efficiency herein is high, preferably at least 5 %, more preferably at least 10 %, 15 %, 20 %, 25 %, 30 %, 35 %, 40 %, 45 %, 50 %, 55 %, 60 %, 65 %, 70 %, even more preferably at least 75 %, 80 %, 85 %, 90 % or 95 %. Thus, in a preferred aspect, the methods for inducing fruiting body formation relate to methods for inducing fruiting body formation at a high induction efficiency herein, preferably at least 5 %, more preferably at least 10 %, 15 %, 20 %, 25%, 30%, 35%, 40%, 45%, 50%, 55 %, 60 %, 65 %, 70 %, even more preferably at least 75 %, 80 %, 85 %, 90 % or 95 % induction efficiency.

The induction efficiency can also be indicated as a fold-increased compared to a method not comprising step (b) of the herein provided method, e.g., where the fungi a incubated at a first predetermined CO₂ concentration (such as when a high CO₂ concentration of between 2500 ppm to 5000 ppm is maintained, reference method). The induction efficiency according to the methods provided herein can be at least 2-fold, 3-fold, 4-fold, 5-fold or 6-fold increased compared to a method not comprising step (b) of the herein provided method.

The x-fold increase can be determined by calculating the induction as explained further above for both the methods provided herein and a method not comprising step (b) of the herein provided method and normalizing the induction efficiency of the herein provided method to that of a method not comprising step (b) of the herein provided method, e.g. where the induction efficiency of the reference method is set to be 1 or 100 %.

### First predetermined CO₂ concentration / Step (a) / (i)

In the methods of the present invention, the first predetermined CO₂ concentration in step a) is above atmospheric levels.

Atmospheric levels of CO₂ are currently at about 400 parts per million (ppm). The skilled person knows that the atmospheric CO₂ levels fluctuate on a daily basis and due to global warming are expected to increase in the coming years.

According to the present invention the first predetermined CO₂ concentration in step a) is above 700 ppm, preferably above 1000 ppm, even more preferably above 1500 ppm, above 2500 ppm and up to 5000 ppm or 10000 ppm. In a particularly preferred embodiment of the present invention, the first predetermined CO₂ concentration in step a) is about 5000 ppm. It is envisaged herein that the first predetermined CO₂ concentration can be a range of the above given concentrations e.g., range from above atmospheric levels to 5000 ppm or 10000 ppm or even higher during the period of cultivation/incubation of step a). For example, a high CO₂ concentration of about 5000 ppm may be reached as follows: incubation starts at atmospheric CO₂ concentration, wherein during incubation a higher than atmospheric CO₂ concentration is reached (e.g., about 5000 ppm), such as due to the increasing metabolization of the fungus. An increase of the CO₂ concentration can readily be obtained by reduced/no ventilation e.g., in closed incubation environments, such as incubators as described above.

It may be useful in the context of the present invention that the incubation in step a) is further performed at a first predetermined temperature and/or a first predetermined humidity.

A useful first predetermined temperature in the methods of the present invention is 18 °C to 30 °C, preferably 22 °C to 28 °C. In a particularly preferred embodiment of the present invention, the first predetermined temperature in step a) is 26 °C.

A useful first predetermined humidity according to the present invention is 35 to 80 %, preferably 40 to 70 %, more preferably 55%. In a particularly preferred embodiment of the present invention, the first predetermined humidity in step a) is 50 %.

In the context of the present invention, it may be particularly useful to further perform the incubation in step a) in the dark or under low light conditions, preferably below 100 lux.

In the context of the present invention, the incubation in step a) is performed until the substrate is fully colonized by the inoculated mycelium. The skilled person will be able to evaluate the timepoint until complete mycelial colonization. In the context of the present invention, this may take typically at least 2 weeks, preferable 2 to 3 weeks. The skilled person will furthermore understand that the incubation is not limited in time, especially, when the incubation is performed at low temperatures. For instance, culture vessels containing fully colonized substrates may be incubated/cultured for up to 6 weeks, before fruiting is induced (i.e., before fruiting body formation is induced by incubating the substrate at a second predetermined CO₂ concentration/step (b)). Likewise, the first predetermined CO₂ concentration will normally reach a peak (e.g., at about 5000 ppm) when the substrate is fully colonized by the inoculated mycelium.

In a particularly preferred embodiment of the present invention, the incubation in step a) is performed up to 20 days.

In the methods of the present invention, the culture conditions, particularly the CO₂ concentration, are/is changed (i.e., decreased, like to a second predetermined CO₂ concentration) when the substrate is fully colonized by the inoculated mycelium.

### Second predetermined CO₂ concentration / Step (b) / (i)

In the methods of the present invention, the second predetermined CO₂ concentration in step b) is lower than the first predetermined CO₂ concentration in step a). For instance, if the first predetermined concentration is above 1500 ppm, the second predetermined determined CO₂ concentration can be any CO₂ concentration, as long as it is lower than 1500 ppm, e.g., 1000 ppm, 700 ppm, and so forth. In other words, the second predetermined CO₂ concentration is relatively lower than the first predetermined CO₂ concentration.

According to the present invention, the second predetermined CO₂ concentration in step b) is below 1500 ppm, preferably below 1000 ppm, more preferably below 700 ppm, even more preferably about atmospheric levels.

In a particularly preferred embodiment of the present invention, the second predetermined CO₂ concentration in step b) is at atmospheric levels.

According to the present invention, incubation at a decreased CO₂ concentration (i.e., corresponding to the second predetermined CO₂ concentration) includes decreasing the CO₂ concentration at the present culture environment or transferring the culture to another culture environment having a decreased CO₂ level. In the context of the present invention, in order to prevent contamination due to change of location, it may be useful to decrease the first predetermined CO₂ concentration to a second predetermined CO₂ concentration at the present culture environment.

In the methods of the present invention, the CO₂ concentration may be decreased (i.e., by increasing ventilation) within 5 days, preferably within 24 hours, preferably within 12 hours.

Herein, it is particularly preferred to decrease the CO₂ concentration within the culture environments within 3 hours.

In the methods of the present invention, the incubation in step b) may be performed at a second predetermined temperature and/or a second predetermined humidity.

It may be particularly useful in the methods for inducing a fruiting body according to the present invention that simultaneously with/after the fruiting body induction (i.e., by incubation at a CO₂ concentration which is decreased compared to the first predetermined CO₂ concentration), the second predetermined temperature is decreased and the second predetermined humidity is increased compared to the first predetermined temperature and humidity, respectively. Similar to the above, the second predetermined temperature/humidity is relatively lower/higher than the first predetermined temperature/humidity, respectively.

A useful second predetermined temperature in the methods of the present invention is 12 °C to 26 °C, preferably 16 °C to 22 °C. In a particularly preferred embodiment of the present invention, the second predetermined temperature in step b) is 18 °C.

A useful second predetermined humidity according to the present invention is above 40 %, preferably above 50 %, more preferably above 70%, even more preferably above 80 %. In a particularly preferred embodiment of the present invention, the second predetermined humidity in step b) is 90 %.

It may be useful in the methods described herein to establish a lighting regimen following the natural day and night rhythm. Typically, the lighting regimen at step b) of the present invention is a 10 to 14 hours lighting regimen at above 100 lux per day, preferably 500 to 3000 lux, per day, but may range from 0 to 3000 lux or can even be higher than 3000 lux.

Generally, it is preferred that a light value is maintained, e.g., when a range of 0 to 3000 lux is mentioned herein, it is preferred that a specific value is chosen, like e.g., 500 lux and this value is maintained for cultivation. However, depending on the development of the fungi /fruiting body, the values may be adapted, e.g., changed from 500 lux to a higher value (and vice versa).
"dark" generally refers preferably to 0 lux, whereas "low light" preferably refers to "1 lux to 100 lux or below". High light preferably refers to more than 100 lux.

It is envisaged herein that the incubation in step b) is further performed at the first predetermined temperature and/or the first predetermined humidity and/or in the dark or under low light conditions. In other words, it is envisaged herein that the temperature and/or humidity and/or dark or under low light conditions used when performing step a) are maintained when performing step (b). These conditions may be further chosen/maintained when performing methods for growing a fruiting body described herein, especially step (ii) thereof. Such conditions may be chosen if it is desired to achieve a more rapid growth of the fruiting body and/or less dense mycelium of the fruiting body and/or a less differentiated fruiting body, such as amorphous fruiting body.

As mentioned above, a useful first predetermined temperature in the methods of the present invention is 18 °C to 30 °C, preferably 22 °C to 28 °C. In a particularly preferred embodiment of the present invention, the first predetermined temperature in step a) is 26 °C. A useful first predetermined humidity according to the present invention is 35 to 80 %, preferably 40 to 70 %, more preferably 55%. In a particularly preferred embodiment of the present invention, the first predetermined humidity in step a) is 50 %. In the context of the present invention, it may be particularly useful to further perform the incubation in step a) in the dark or under low light conditions, preferably below 100 lux.

The skilled person will understand that successful fruiting herein can be visually determined by development/appearance of primordia.

The incubation in step b) is performed under conditions allowing the induction of fruiting bodies and/or until fruiting bodies (primordia) have formed. The skilled person will understand that the fruiting (primordia formation) may vary in time. In the context of the present invention, the fruiting body induction (specifically in step b)) typically takes up to 7 days.

### Growing Method

In a second aspect, the present invention relates to a method for growing a fruiting body of a fungus whose induction is triggered by a decrease in the CO₂ concentration during the culture process.

The method of the second aspect of the present invention comprises the following steps:
i) inducing fruiting body formation according to the methods for inducing fruiting body formation of the present invention;
ii) incubating the substrate until a fruiting body has grown;
iii) harvesting the fruiting body.

The skilled person will understand that the fruiting (primordia formation) may vary in time. In the context of the present invention, the fruiting body induction in step i) typically takes up to 7 days.

In the methods of the present invention, the incubation in step ii) is performed at the second predetermined CO₂ concentration and may be further performed at a second predetermined temperature and/or a second predetermined humidity.

It may be particularly useful in the methods for growing a fruiting body according to the present invention that after the fruiting body induction, the second predetermined temperature is decreased and the second predetermined humidity is increased compared to the first predetermined temperature and humidity, respectively. Similar to the above, the second predetermined temperature/humidity is relatively lower/higher than the first predetermined temperature/humidity, respectively. The same explanations apply to the light conditions.

Alternatively, in order to simplify the culture process and already discussed above, the second predetermined temperature and/or the second predetermined humidity and/or light condition can already be adjusted simultaneously with the decrease of the CO₂ concentration, i.e., during step b) in the aforementioned induction method. In this case, the predetermined temperature and/or the second predetermined humidity and/or light condition are/is maintained throughout the growth phase of the mushroom (i.e., during the incubation in step ii)).

A useful second predetermined temperature in the methods of the present invention is 12 °C to 26 °C, preferably 16 °C to 22 °C. In a particularly preferred embodiment of the present invention, the second predetermined temperature in step ii) is 18 °C.

A useful second predetermined humidity according to the present invention is above 40 %, preferably above 50 %, more preferably above 70%, even more preferably above 80 %. In a particularly preferred embodiment of the present invention, the second predetermined humidity in step ii) is 90 %.

In order to closer mimic mushroom growth conditions in the wild, it may be useful in the growing methods described herein to establish a lighting regimen following the natural day and night rhythm. Typically, the lighting regimen at step ii) of the present invention is a 10 to 14 hours lighting regimen at 500 to 3000 lux per day.

It is envisaged herein that the incubation in step b) and/or step ii) is further performed at the first predetermined temperature and/or the first predetermined humidity and/or in the dark or under low light conditions. In other words, it is envisaged herein that the temperature and/or humidity and/or dark or under low light conditions used when performing step a) are maintained when performing step (b) and/or step (ii). These conditions may be further chosen/maintained when performing methods for growing a fruiting body described herein, especially step (ii) thereof. Such conditions may be chosen if it is desired to achieve a more rapid growth of the fruiting body and/or less dense mycelium of the fruiting body and/or a less differentiated fruiting body, such as amorphous fruiting body.

As mentioned above, a useful first predetermined temperature in the methods of the present invention is 18 °C to 30 °C, preferably 22 °C to 28 °C. In a particularly preferred embodiment of the present invention, the first predetermined temperature in step a) is 26 °C. A useful first predetermined humidity according to the present invention is 35 to 80 %, preferably 40 to 70 %, more preferably 55%. In a particularly preferred embodiment of the present invention, the first predetermined humidity in step a) is 50 %. In the context of the present invention, it may be particularly useful to further perform the incubation in step a) in the dark or under low light conditions, preferably below 100 lux.

The skilled person is able to determine when a fruiting body of a mushroom is ready to harvest. The skilled person will understand that, in the context of the present invention, young fruiting bodies, i.e., fruiting bodies before sporing are to be harvested.

As mentioned, in the context of the present invention, "fruiting body" preferably refers to the mature/fully differentiated stage of mushroom. The skilled person will understand that in the context of the present invention, fruiting bodies are usually the fleshy consumed/consumable part of edible fungal species; the fruiting bodies typically consist of a dense/compact network of the mycelial matter of the fungus and/or are the mature/fully differentiated stage of mushroom and/or are larger in size compared to the above mentioned primordia (i.e. compared to the initial stage of fruiting body formation).Also developing, immature fruiting bodies may be consumed, as it is custom with other fungi species. Accordingly, in the context of the present invention, "fruiting body" can also refer to developing and/or immature fruiting bodies. "Developing and/or immature fruiting body" in this context refers primarily to amorphous, developing and/or immature fruiting body (also termed herein amorphous fruiting body or amorphous immature fruiting body or amorphous developing fruiting body), e.g., fruiting bodies grown e.g., in the dark or under low light conditions (preferably < 100 lux), e.g., when performing step (ii) of the methods provided herein. "Developing and/or immature fruiting body" are usually larger in size compared to the primordia which are the initial, immature stage of the mushroom's fruiting bodies, as explained above. For example, a "developing and/or immature fruiting body" can have a diameter of above 7.5 mm and up to several cm, e.g., up to 10, 15 or 20 cm). Without deferring from the gist of invention, it is contemplated that (a sample of) "fruiting bodies" (e.g., to be harvested in accordance with the provided methods) as defined herein can, in addition, comprise primordia (in the sense of initial, immature stage of the mushroom's fruiting bodies or parts thereof). Preferably, (a sample of) "fruiting bodies" (e.g., to be harvested in accordance with the provided methods) as defined herein does not comprise primordia (in the sense of initial, immature stage of the mushroom's fruiting bodies or parts thereof).

A "fruiting body" herein preferably is a fresh weight fruiting body, i.e., a freshly harvested fruiting body (e.g., from shortly after harvest (like several minutes) to up to several weeks, particularly when stored at low temperatures (e.g., at 4 °C)), as compared to a dry/dried fruiting body. Most preferably, the "fruiting body" is pasteurized (directly) after harvest. Such a pasteurized fruiting body can then be stored for up to several weeks, particularly when stored at low temperatures (e.g., at 4 °C).

In the context of the present invention, the incubation in step ii) is performed for approximately 7 to 14 days. In other words, typically the cultured fruiting bodies described herein are ready to harvest approximately 7 to 14 days after primordia development.

In the methods of the present invention, the harvest in step iii) may be performed by any means suitable for harvesting fruiting bodies known in the art, including but not limited to, harvesting by cutting and the like.

Mushrooms/Fungi derive all the energy and factors necessary for growth through biochemical decomposition processes from the substrate on which they grow. Consequently, all the factors required for fruiting and subsequent growth of the fruiting bodies must already be present in the substrate.

However, since also potentially harmful ingredients may be adsorbed from the growing mushrooms, the substrates according to the present invention are preferably free of toxins and/or substances rendering the fruiting bodies deleterious or inedible or otherwise unsuitable for human consumption (e.g., when the fruiting bodies are not in food-grade quality).

### Substrate

In a third aspect, the present invention relates to substrates which may be used for inducing and growing a fruiting body of a fungus in the context of the present invention.

The substrate according to the present invention comprises or consists of the following components:
a) a cellulose component;
b) an amino acid component; and
c) a mineral component;
d) and, optionally, a fibrous component.

The substrate can, in addition/optionally, contain further components, such as a saccharated component, e.g., sugar, e.g., dextrose.

It goes without saying that the amount/percentage of the components should be chosen to sum up to 100 % of the substrate, e.g., when the amount/percentage of the component(s) above is indicated with reference to the total dry weight of the substrate.

For example, when the substrate comprises an amino acid component of 41.2 %, the remaining components are composed of 58.8 % dry weight of the substrate. This may be the case when the substrate is a "dry" substrate, i.e., does not comprise water or at least essentially no water. This kind of substrate can be prepared and stored; before using the substrate for fungus cultivation, an appropriate amount of water (or "predetermined" amount/percentage of water) needs to be added. The substrate with water should be sterilized prior to use and/or used in suitable (sterile) culture vessels, see below.

These explanations also apply when the substrate comprises a predetermined amount/percentage of water (like a "ready to use" substrate). In that case the indicated amount/percentage of the components refers to the amount/percentage of the dry weight of the substrate (i.e., the amount/percentage of the substrate without water).

When the substrate comprises a predetermined amount/percentage of water, the amount/percentage of water is indicated with reference to all other components of the substrate (e.g., cellulose component, amino acid component, mineral component, and, optionally, a fibrous component and/or a carbohydrate/sugar, e.g., dextrose). For example, a water content of 43 % means the substrate comprises 43 % water and 57 % other components.

When the amount/percentage of the components, e.g., a cellulose component, an amino acid component, a mineral component, a fibrous component and/or further components, such as a carbohydrate/sugar, e.g., dextrose, is indicated herein as "% of the total dry weight of the substrate" it refers to the amount/percentage (w/w) of the (dry) component relative to the total amount/percentage of the (dry) substrate.

When the substrate comprises a predetermined amount/percentage of water, the amount/percentage of water is indicated as "% water content" it refers to the amount/percentage (w/w) of water relative to the total amount/percentage of the (wet) substrate.

### Cellulose component

In the context of the present invention, the cellulose component is selected from the group consisting of wood chips, saw dust and/or combinations thereof.

The wood chips and/or saw dust useful according to the present invention can be derived from deciduous trees and/or coniferous trees.

"Derived from" as used herein means the cellulose component (wood chips, saw dust and/or combinations thereof) is a plant cellulose component, specifically a wood/tree cellulose component. For example, wood chips and/or saw dust derived from deciduous trees is/are a deciduous tree wood chips and/or saw dust. As another example, wood chips and/or saw dust derived from beech is/are beech wood chips and/or beech saw dust. This explanation applies mutatis mutandis to other aspects herein, e.g., exemplary trees mentioned herein below.

In a preferred embodiment the wood chips and/or saw dust are derived from trees selected from the group consisting of alder, ash, beech, birch, fruit trees (e.g., apple, pear, plum), poplar, willow, fir, larch, pine, and/or combinations thereof.

In a particularly preferred embodiment of the present invention, the wooden component is derived from beech.

In the context of the present invention, in order to produce fruiting bodies with food-grade quality, the cellulose component contained in the substrates used herein is particularly not derived from timbers containing toxins such as yew, robinia and the like and/or containing a high amount of tannic acid such as oak, chestnut and the like.

In the context of the present invention, the size of the wood chips is not particularly limited and can be any suitable size known in the art.

The size of the wood chips for use in the present invention is preferably between 0.2 and 30 mm.

Typically, the amount of the cellulose component in the substrates of the present invention is between 25 % to 75 % of the total dry weight of the substrate, preferably between 35 to 65 % of the total dry weight of the substrate, more preferably between 45 to 55 % of the total dry weight of the substrate.

It is particularly preferred that the cellulose component in the substrate of the present invention is a mixture of wood chips and saw dust derived from beech, preferably amounting to about 47 % of the total dry weight of the substrate.

### Amino acid component

For optimal growth conditions and production of fruiting bodies with high nutritional value, the substrates used herein comprise an amino acid component, preferably a plant-based amino acid component.

In the context of the present invention, the amino acid component may be selected from the group consisting of grains, bran, malt, meal, algae, dried herbal matter (e.g., alfalfa hay), spirulina powder, nutritional yeast (e.g., beer yeast), nuts, beans, legumes and/or combinations thereof.

Grains useful as amino acid component in the substrates of the present invention are not particularly limited and can be any cereal/kernel suitable for human consumption known in the art, including wheat, rye, rice, millet, barley, oats, corn, buckwheat, quinoa, chia, and the like.

Bran (i.e., miller's bran) comprises the outer shells of grains. In the context of the present invention, bran may contain the outer shell of any one of the aforementioned types of grain and/or mixtures thereof.

Malt is germinated grain dried in a process known as malting. In the context of the present invention, malt may be based on any one of the aforementioned types of grain, such as barley malt, and/or mixtures thereof.

Typically, meal may comprise residues of oil production in form of ground, defatted seeds, including but not limited to, lin, pumpkin, coco, poppy, rape, sunflower, sesame, thistle, soy, palm fruits (e.g., saw palmetto fruits, açaí berry) and other seeds used for oil production (e.g., hemp seeds) known in the art.

According to the present invention, the amount of the amino acid component in the substrates of the present invention is between 1 % to 50 % of the total dry weight of the substrate, preferably between 8 % to 47.5 %, more preferably 35 to 47 %, and even more preferably 40 to 45 % of the total dry weight of the substrate.

It is particularly preferred that the amino acid component in the substrate of the present invention is amounting to about 41 % (e.g., 41.2 % or 41.3 %) of the total dry weight of the substrate. The amino acid component preferably is a mixture of wheat berries, wheat bran, corn flour and (deoiled) lin meal (preferably amounting to about 9.4 %, about 23.4 %, about 6.1 % and about 2.3 % of the total dry weight of the substrate, respectively).

The skilled person will understand that the amino acid component according to the present invention does not need to exclusively contain amino acids (e.g., in form of single amino acids, peptides (e.g., 2 to 20 amino acids in length) and/or primarily in form of proteins (e.g., more than 20 and up to 200 or more amino acids in length)) and can contain in addition other compounds e.g., carbohydrates. Such other compounds can be advantageous for the mushroom to be cultured.

### Mineral component

It is common general knowledge in the art that mycelial growth is accompanied by a decrease of the pH in the substrate. This is so, because the growing mycelium excretes increasing amounts of organic acids including, inter alia, oxalic acid.

In order to counteract the aforementioned acidification of the substrate (typically, decrease from pH 7 to a pH between 3 to 4), buffers stabilizing the pH may be added to the substrates used herein. In the context of the present invention, any buffer known in the art which is not contrary to (inter)national food safety and quality laws and regulations, such as EDTA, may be used to optimize (i.e., increase) the pH of the substrates. An increase in the pH may additionally prevent the growth of mold and bacteria on top of and/or in the substrate.

Typically, the substrates used herein comprise a mineral component acting as a pH buffer.

In the context of the present invention, the mineral component in the substrate can be selected from the group consisting of chalk, calcium carbonate (CaCO₃), dolomite, gypsum, ground seashells, coralligene, bone and/or other natural sources of calcium carbonate, magnesium carbonate, calcium sulphate and potassium sulphate and/or combinations thereof. In the context of the present invention, dolomite refers to a mixture of CaCO₃ and MgCO₃.

Particularly, in order to produce fruiting bodies with food-grade quality, the mineral component contained in the substrates of the present invention is not toxic or renders the produced fruiting-bodies inedible, such as Zinc sulfate (ZnSO₄) and the like. Accordingly, the substrate provided herein does preferably not comprise or contain a mineral component which is toxic or renders the produced fruiting-bodies inedible, such as Zinc sulfate (ZnSO₄).

According to the present invention, the amount of the mineral component in the substrates of the present invention is between 1 % to 10 % of the total dry weight of the substrate, preferably between 2.5 % to 7.5 % of the total dry weight of the substrate, more preferably between 4.5 to 5.5 % of the total dry weight of the substrate, most preferably is 5.4 % of the total dry weight of the substrate.

It is particularly preferred that the mineral component in the substrate of the present invention is a mixture of CaCO₃, gypsum and dolomite, preferably amounting to 0.9 %, 3.1 % and 1.4 % of the total dry weight of the substrate, respectively.

### Fibrous component

In order to enhance aeration of the substrate and consequently, oxygen supply of the mycelium, the substrates of the present invention may contain a fibrous component.

For reasons of sustainability, the fibrous component to be used in the present invention may be generated as by-product and/or waste product of agriculture, forestry and the like.

Herein, the fibrous component is plant-based and as such can include any vegetable-based fiber, wood-based fiber or stem material of low density.

According to the present invention, the fibrous component is preferably selected from the group consisting of hemp, wood fiber, peat, paper or cardboard, cotton hulls, jute, banana, coco, bagasse, flax, reed, nettle, straw, hay and/or combinations thereof.

According to the present invention, the amount of the fibrous component in the substrates of the present invention is between 2 % to 45 % of the total dry weight of the substrate, preferably between 4 % to 30 % of the total dry weight of the substrate, more preferably between 5 to 25 % of the total dry weight of the substrate, even more preferably between 5 to 10 % of the total dry weight of the substrate.

In a particularly preferred embodiment of the present invention, the fibrous component is hemp, preferably amounting to 5.6 % of the total dry weight of the substrate.

### Water

In order to prevent drying out and atrophy of the mushrooms, the substrates used herein typically have a predetermined water content, i.e., the aforementioned dry substrate ingredients are mixed with water to obtain a wet substrate with an adjusted amount of water.

The predetermined water content of the substrate in the context of the present invention is preferably between 30 % to 70 %, preferably between 35 % to 55 %, more preferably between 40 % to 50 %, even more preferably between 40 % to 45 %.

In a particularly preferred embodiment of the present invention, the predetermined water content of the substrate is about 43 % (e.g., 42.8 %).

According to the present invention, the term "water" refers to water having drinking water quality, including but not limited to, tab water, deionized water, distilled water, autoclaved water and the like. In the context of the present invention, deionized water is particularly useful.

### Saccharated component

In order to accelerate the colonization process of the substrate by the mycelium, i.e., the (initial) mycelial growth phase, the substrate used herein may further be supplemented with a saccharated component.

In the context of the present invention, the saccharated component is selected from the group consisting of glucose, fructose, galactose, dextrose, maltose, sucrose, lactose, polyalcohols, glycosides, N-acetylglucosamine and/or combinations thereof.

Typically, the amount of the saccharated component in the substrates of the present invention is between 0 % to 5 % of the total dry weight of the substrate, preferably between 0.5 % to 2 % of the total dry weight of the substrate, more preferably between 0.8 to 1.5 % of the total dry weight of the substrate.

Particularly, the saccharated component present in the substrates of the present invention is dextrose, preferably amounting to 0.9 % of the total dry weight of the substrate.

The following relates to preferred substrates to be used and provided in the present invention.

In a preferred embodiment, the substrate according to the present invention comprises or consists of the following components:
a) a cellulose component, wherein the cellulose component is a mixture of wood chips and saw dust derived from beech (i.e. beech wood chips and beech saw dust), preferably amounting to about 46 % or 47 % (e.g. preferably 46.8 %) of the total dry weight of the substrate, more preferably wherein the wood chips amounting to about 38 % (e.g. preferably 38.4 %-38.5 %, more preferably 38.5 %) of the total dry weight of the substrate and the saw dust amounts to about 8 % (e.g. preferably 8.4 %) of the total dry weight of the substrate;
b) an amino acid component amounting to about 41 % (e.g., preferably 41.2 % (or 41.3 %)) of the total dry weight of the substrate, wherein the amino acid component preferably is a mixture of wheat berries, wheat bran, corn flour and (deoiled) lin meal (preferably amounting to about 9.4 %, about 23.4 %, about 6.1 % and about 2.3 % of the total dry weight of the substrate, respectively),
c) a mineral component amounting to about 5 % (e.g. preferably 5.4 %) of the total dry weight of the substrate, more preferably wherein the mineral component is a mixture of CaCO₃, gypsum and dolomite, preferably amounting to 0.9 %, 3.1 % and 1.4 % of the total dry weight of the substrate, respectively,
d) a fibrous component amounting to about 5 % (e.g. preferably 5.6 %) of the total dry weight of the substrate, more preferably wherein the fibrous component is hemp/hemp fiber, preferably amounting to about 5 % (e.g. preferably 5.6 %) of the total dry weight of the substrate;
e) a saccharated component amounting to about 1 % (e.g. preferably 0.9 %), preferably wherein the saccharated component is dextrose, preferably amounting to about 1 % (e.g. preferably 0.9 %) of the total dry weight of the substrate.

In a further preferred embodiment, the substrate according to the present invention (particularly as defined hereinabove) comprises a predetermined water content, preferably between 30 % to 70 %, preferably between 35 % to 55 %, more preferably about 35 %, about 43 %, about 55 %, or about 60 %. Most preferably, the substrate according to the present invention comprises a predetermined water content of about 43 %, particularly preferred 42.8 %).

In a particularly preferred embodiment, the substrate according to the present invention comprises or consists of the following components:
2000 g wheat berries, 1800 g beech saw dust, 8200 g beech wood chips (between 5 and 20 mm) and 1200 g hemp fiber, 16000 g water, 5000 g wheat bran, 1300 g corn flour, 670 g gypsum, 300 g Dolomite, 200 g CaCO₃, 200 g dextrose and 500 g (deoiled) lin meal.

In further preferred embodiments the substrate comprises instead of 16000 g water: 11500 g water, 26000 g water or 32500 g water.

The substrate above can be prepared as follows: 2000 g wheat berries, 1800 g beech saw dust, 8200 g beech wood chips (between 5 and 20 mm) and 1200 g hemp fiber are mixed and soaked in 16000 g water for 12-16 h. 5000 g wheat bran, 1300 g corn flour, 670 g gypsum, 300 g Dolomite, 200 g CaCO₃, 200 g dextrose and 500 g lin meal are mixed dry and added to the soaked wood chips. Everything is then mixed together until all ingredients are blended well.

In the following, the amount of the mentioned substrate components is indicated per dry weight (DW) as well as per wet substrate (WS). The amount per wet substrate (WS) is indicated in brackets.

Examples 3 to 16, respectively, provide substrates with concrete amounts corresponding to the relative values given in the embodiments hereinbelow. The order of the embodiments herein below follows the order of the Examples 3 to 16. Accordingly, in the place of the embodiments hereinbelow also concrete substrates with concrete amounts provides in Examples 3 to 16 can be used in the general context of the present invention. This has been done exemplarily herein for the preferred substrates provided/used in Examples 3 to 6, wherein the substrate of Example 3 is particularly preferred. The same can, mutatis mutandis, be done for the substrates provided/used in Examples 7 to 16.

In a preferred embodiment, the present invention relates to a substrate which comprises:
a) a cellulose component consisting of 8.4 % DW (4.8 % WS) beech saw dust and 38.4 to 38.5 % DW, preferably 38.5 % DW (21.9 % WS) beech wood chips;
b) an amino acid component consisting of 9.4 % DW (5.4 % WS) wheat berries, 23.4 % DW (13.4 % WS) wheat bran, 6.1 % DW (3.5 % WS) corn flour and 2.3 % DW (1.3 % WS) lin meal (deoilded);
c) a mineral component consisting of 3.1 % DW (1.8 % WS) gypsum, 1.4 % DW (0.8 % WS) dolomite and 0.9 % DW (0.5 % WS) CaCO₃;
d) a fibrous component consisting of 5.6 % DW (3.2 % WS) hemp fiber;
e) a saccharated component consisting of 0.9 % DW (0.5 % WS) dextrose;
and a predetermined water content of about 43 %.

In another preferred embodiment, the present invention relates to a substrate which comprises:
a) a cellulose component consisting of 8.4 % DW (4.8 % WS) beech saw dust and 38.4 % to 38.5 % DW, preferably 38.5 % DW (21.9 % WS) beech wood chips;
b) an amino acid component consisting of 9.4 % DW (5.4 % WS) wheat berries, 23.4 % DW (13.4 % WS) wheat bran, 6.1 % DW (3.5 % WS) corn flour and 2.3 % DW (1.3 % WS) lin meal (deoilded);
c) a mineral component consisting of 3.1 % DW gypsum (2.0 % WS), 1.4 % DW (0.9 % WS) dolomite and 0.9 % DW (0.6 % WS) CaCO₃;
d) a fibrous component consisting of 5.6 % DW (3.2 % WS) hemp fiber;
e) a saccharated component consisting of 0.9 % DW (0.6 % WS) dextrose;
and a predetermined water content of about 31 %.

In another preferred embodiment, the present invention relates to a substrate which comprises:
a) a cellulose component consisting of 8.4 % DW (4.8 % WS) beech saw dust and 38.4 % to 38.5 % DW, preferably 38.5 % DW (17.3 % WS) beech wood chips;
b) an amino acid component consisting of 9.4 % DW (4.2 % WS) wheat berries, 23.4 % DW (10.6 % WS) wheat bran, 6.1 % DW (2.7 % WS) corn flour and 2.3 % DW (1.1 % WS) lin meal (deoilded);
c) a mineral component consisting of 3.1 % DW (1.4 % WS) gypsum, 1.4 % DW (0.6 % WS) dolomite and 0.9 % DW (0.4 % WS) CaCO₃;
d) a fibrous component consisting of 5.6 % DW (2.5 % WS) hemp fiber;
e) a saccharated component consisting of 0.9 % DW (0.4 % WS) dextrose;
and a predetermined water content of about 55 %.

In another preferred embodiment, the present invention relates to a substrate which comprises:
a) a cellulose component consisting of 8.4 % DW (3.3 % WS) beech saw dust and 38.4 % to 38.5 % DW, preferably 38.5 % DW (15.2 % WS) beech wood chips;
b) an amino acid component consisting of 9.4 % DW (3.7 % WS) wheat berries, 23.4 % DW (9.3 % WS) wheat bran, 6.1 % DW (2.4 % WS) corn flour and 2.3 % DW (0.9 % WS) lin meal (deoilded);
c) a mineral component consisting of 3.1 % DW (1.2 % WS) gypsum, 1.4 % DW (0.6 % WS) dolomite and 0.9 % DW (0.4 % WS) CaCO₃;
d) a fibrous component consisting of 5.6 % DW (2.2 % WS) hemp fiber;
e) a saccharated component consisting of 0.9 % DW (0.4 % WS) dextrose;
and a predetermined water content of about 60 %.

In another preferred embodiment, the present invention relates to a substrate which comprises:
a) a cellulose component consisting of 8.4 % DW (4.8 % WS) beech saw dust and 38.4 % DW (21.9 % WS) beech wood chips;
b) an amino acid component consisting of 9.4 % DW (5.4 % WS) wheat berries, 6.1 % DW (3.5 % WS) corn flour, 4.7 % DW (2.7 % WS) sunflower meal, 4.7 % DW (2.7 % WS) poppy seed meal, 4.7 % DW (2.7 % WS) hemp meal, 4.7 % DW (2.7 % WS) pumpkin seed meal, 4.7 % DW (2.7 % WS) rape meal and 2.3 % DW (1.3 % WS) lin meal (deoilded);
c) a mineral component consisting of 3.1 % DW (1.8 % WS) gypsum, 1.4 % DW (0.8 % WS) dolomite and 0.9 % DW (0.5 % WS) CaCO₃;
d) a fibrous component consisting of 5.6 % (3.2 % WS) DW hemp fiber;
e) a saccharated component consisting of 0.9 % DW (0.5 % WS) dextrose;
and a predetermined water content of about 43 %.

In another preferred embodiment, the present invention relates to a substrate which comprises:
a) a cellulose component consisting of 8.4 % DW (4.8 % WS) beech saw dust and 38.4 % to 38.5 % DW, preferably 38.5 % DW (21.9 % WS) beech wood chips;
b) an amino acid component consisting of 9.4 % DW (5.4 % WS) wheat berries, 6.1 % DW (3.5 % WS) corn flour, 23.4 % DW (13.4 % WS) sunflower meal and 2.3 % DW (1.3 % WS) lin meal (deoilded);
c) a mineral component consisting of 3.1 % DW (1.8 % WS) gypsum, 1.4 % DW (0.8 % WS) dolomite and 0.9 % DW (0.5 % WS) CaCO₃;
d) a fibrous component consisting of 5.6 % DW (3.2 % WS) hemp fiber;
e) a saccharated component consisting of 0.9 % DW (0.5 % WS) dextrose;
and a predetermined water content of about 43 %.

In another preferred embodiment, the present invention relates to a substrate which comprises:
a) a cellulose component consisting of 8.4 % DW (4.8 % WS) beech saw dust and 38.4 % to 38.5 % DW, preferably 38.5 % DW (21.9 % WS) beech wood chips;
b) an amino acid component consisting of 9.4 % DW (5.4 % WS) wheat berries, 6.1 % DW (3.5 % WS) corn flour, 23.4 % DW (13.4 % WS) beer yeast and 2.3 % DW (1.3 % WS) lin meal (deoilded);
c) a mineral component consisting of 3.1 % DW (1.8 % WS) gypsum, 1.4 % DW (0.8 % WS) dolomite and 0.9 % DW (0.5 % WS) CaCO₃;
d) a fibrous component consisting of 5.6 % DW (3.2 % WS) hemp fiber;
e) a saccharated component consisting of 0.9 % DW (0.5 % WS) dextrose;
and a predetermined water content of about 43 %.

In another preferred embodiment, the present invention relates to a substrate which comprises:
a) a cellulose component consisting of 8.4 % DW (4.8 % WS) beech saw dust and 19.7 % DW (11.2 % WS) beech wood chips;
b) an amino acid component consisting of 9.4 % DW (5.4 % WS) wheat berries, 23.4 % DW (13.4 % WS) wheat bran, 6.1 % DW (3.5 % WS) corn flour and 2.3 % DW (1.3 % WS) lin meal (deoilded);
c) a mineral component consisting of 3.1 % DW (1.8 % WS) gypsum, 1.4 % DW (0.8 % WS) dolomite and 0.9 % DW (0.5 % WS) CaCO₃;
d) a fibrous component consisting of 5.6 % DW (3.2 % WS) hemp fiber, 9.4 % DW (5.4 % WS) lin fiber and 9.4 % DW (5.4 % WS) straw (cut);
e) a saccharated component consisting of 0.9 % DW (0.5 % WS) dextrose;
and a predetermined water content of about 43 %.

In another preferred embodiment, the present invention relates to a substrate which comprises:
a) a cellulose component consisting of 14.3 % DW (8.1 % WS) beech saw dust and 57.2 % DW (32.5 % WS) beech wood chips;
b) an amino acid component consisting of 6.2 % DW (3.5 % WS) wheat bran and 2.4 % DW (1.4 % WS) lin meal (deoilded);
c) a mineral component consisting of 3.2 % DW (1.8 % WS) gypsum, 1.4 % DW (0.8 % WS) dolomite and 1.0 % DW (0.5 % WS) CaCO₃;
d) a fibrous component consisting of 14.3 % DW (8.1 % WS) hemp fiber;
and a predetermined water content of about 43 %.

In another preferred embodiment, the present invention relates to a substrate which comprises:
a) a cellulose component consisting of 7.5 % DW (4.3 % WS) beech saw dust and 34.4 % to 34.5 % DW, preferably 34.5 % DW (19.6 % WS) beech wood chips;
b) an amino acid component consisting of 8.4 % DW (4.8 % WS) wheat berries, 20.9 % DW (11.9 % WS) wheat bran, 5.4 % DW (3.1 % WS) corn flour and 12.6 % DW (7.2 % WS) lin meal (deoilded);
c) a mineral component consisting of 2.8 % DW (1.6 % WS) gypsum, 1.3 % DW (0.7 % WS) dolomite and 0.8 % DW (0.5 % WS) CaCO₃;
d) a fibrous component consisting of 5.0 % DW (2.9 % WS) hemp fiber;
e) a saccharated component consisting of 0.8 % DW (0.5 % WS) dextrose;
and a predetermined water content of about 43 %.

In another preferred embodiment, the present invention relates to a substrate which comprises:
a) a cellulose component consisting of 8.4 % DW (4.8 % WS) beech saw dust and 44.0 % to 44.1 % DW, preferably 44.1 % DW (25.2 % WS) beech wood chips;
b) an amino acid component consisting of 9.4 % DW (5.5 % WS) wheat berries, 23.4 % DW (13.4 % WS) wheat bran, 6.1 % DW (3.5 % WS) corn flour and 2.3 % DW (1.3 % WS) lin meal (deoilded);
c) a mineral component consisting of 3.1 % DW (1.8 % WS) gypsum, 1.4 % DW (0.8 % WS) dolomite and 0.9 % DW (0.5 % WS) CaCO₃;
e) a saccharated component consisting of 0.9 % DW (0.5 % WS) dextrose;
and a predetermined water content of about 43 %.

In another preferred embodiment, the present invention relates to a substrate which comprises:
a) a cellulose component consisting of 8.4 % DW (4.8 % WS) beech saw dust and 38.4 % to 38.5 % DW, preferably 38.5 % DW (21.9 % WS) beech wood chips;
b) an amino acid component consisting of 9.4 % DW (5.4 % WS) wheat berries, 6.1 % DW (3.5 % WS) corn flour and 25.7 % DW (14.7 % WS) rape meal;
c) a mineral component consisting of 3.1 % DW (1.8 % WS) gypsum, 1.4 % DW (0.8 % WS) dolomite and 0.9 % DW (0.5 % WS) CaCO₃;
d) a fibrous component consisting of 5.6 % DW (3.2 % WS) hemp fiber;
e) a saccharated component consisting of 0.9 % DW (0.5 % WS) dextrose;
and a predetermined water content of about 43 %.

In another preferred embodiment, the present invention relates to a substrate which comprises:
a) a cellulose component consisting of 8.5 % DW (4.9 % WS) beech saw dust and 38.9 % to 39.1 % DW, preferably 39.1 % DW (22.1 % WS) beech wood chips;
b) an amino acid component consisting of 9.5 % DW (5.4 % WS) wheat berries, 4.7 % DW (2.7 % WS) wheat bran, 23.7 % DW (13.5 % WS) corn flour and 2.4 % DW (1.3 % WS) lin meal (deoilded);
c) a mineral component consisting of 3.2 % DW (1.8 % WS) gypsum, 1.4 % DW (0.8 % WS) dolomite and 0.9 % DW (0.5 % WS) CaCO₃;
d) a fibrous component consisting of 5.7 % DW (3.2 % WS) hemp fiber;
e) a saccharated component consisting of 0.9 % DW (0.5 % WS) dextrose;
and a predetermined water content of about 43 %.

In another preferred embodiment, the present invention relates to a substrate which comprises:
a) a cellulose component consisting of 8.4 % DW (4.8 % WS) beech saw dust;
b) an amino acid component consisting of 9.4 % DW (5.4 % WS) wheat berries, 23.4 % DW (13.4 % WS) wheat bran, 6.1 % DW (3.5 % WS) corn flour and 2.3 % DW (1.3 % WS) lin meal (deoilded);
c) a mineral component consisting of 3.1 % DW (1.8 % WS) gypsum, 1.4 % DW (0.8 % WS) dolomite and 0.9 % DW (0.5 % WS) CaCO₃;
d) a fibrous component consisting of 44.0 % to 44.1 % DW, preferably 44.1 % DW (25.2 % WS) hemp fiber;
e) a saccharated component consisting of 0.9 % DW (0.5 % WS) dextrose;
and a predetermined water content of about 43 %.

### Sterilization of the Substrate

Substrate can be easily contaminated with airborne microorganisms interfering with mushroom growth and ultimately, decreasing the yield. Thus, besides the requirement of the ideal growth conditions for the destined mushroom species, contamination with other fungi and bacteria must be prevented.

Mushrooms of the species *Laetiporus sulphureus* are reported to be sensible for contamination, particularly with fungi from the genera Penicillium and Trichoderma [NPL16].

As the growth conditions suitable for the production of edible mushrooms may be also ideal for many contaminants, the present invention relates to the use of sterile substrates and any means suitable for sterilizing known in the art, including steam sterilization (e.g., autoclaving), chemical sterilization, pasteurizing and the like may be employed. In the context of the present invention, steam sterilization is preferred.

In the context of the present invention, the sterilizing step is performed at temperatures sufficiently high and times sufficiently long to inactivate/kill undesired microorganism. Herein, it may be particularly useful to sterilize at temperatures between 80 °C to 135 °C for at least 1 hour, preferably at least 2 to 3 hours, more preferably at least 4 hours. In a particularly useful embodiment of the present invention the substrate is sterilized at 98 °C for 3.5 hours.

### Culture Vessels

The skilled person will understand that in order to sterilize the substrates used in the present invention, said substrates may be provided in sterilizable vessels, preferably hermetically sealed sterilizable vessels, including but not limited to, polymer bags (e.g., polypropylene bags), polymer and glass containers and the like.

It is one advantage over the prior art of the herein provided methods that the culture vessels (containing sterile substrate, see above) to be used herein need not be opened during induction and/or growth of the fruiting bodies, i.e., the substrate in the culture vessels to be used herein remains sterile throughout performing the methods herein, e.g., until after the fruiting body is harvested. As explained, some prior art methods require opening the culture vessels to induce fruiting body formation by watering the substrate. This can lead to contamination of the substrate which can be avoided by applying/using the methods herein.

These sterilizable vessels may further contain one or more filters with a defined mesh size. The filter used herein may have a mesh size which is sufficiently big to enable the outgrowth of the hyphae but also sufficiently small to prevent permeation of potential sources of contamination, including, inter alia, bacteria, dust particles, spores of other fungi and the like.

In the context of the present invention, the filter has a mesh size of 2 µm to 50 µm, preferably of 2 µm to 20 µm, more preferably of 5 µm.

In a particularly preferred embodiment of the present invention, the substrate is filled in polypropylene bags with a 5 µm filter and subsequently sterilized.

The skilled person knows that the amount of substrate to be filled in the culture vessels useful in the context of the present invention depends on the capacity of the culture vessel. It is pointed out that the skilled person will be able to determine the amount of substrate to be filled into the culture vessel. The amount of substrate to be filled into the culture vessel used herein may be sufficiently small to enable complete closure of the culture vessel. According to the present invention, typically 3 kilograms of substrate are to be filled into the culture vessel.

Typically, after being sterilized, the substrate of the present invention is cooled down to temperatures between 20 °C to 35 °C, preferably 25 °C to 30 °C. In a preferred embodiment the substrate after sterilization is cooled down to 28 °C prior to being inoculated with spawn.

In order to maintain sterile conditions with respect to the methods described herein, steps which involve opening of the culture vessels after sterilization, e.g., the inoculation of the substrate with spawn, have to be performed in sterile environments, including but not limited to laminar flow cabins, clean rooms and the like. In a preferred embodiment, inoculation is performed in a laminar flow cabin.

The skilled person will further understand that any additional steps which might cause the loss of sterility should be avoided.

In the context of the present invention, the sterile substrate is to be inoculation with the spawn. The person skilled in the art will be able to determine the amount of spawn to be inoculated into the substrate. Filled sterile substrates useful according to the present invention are typically inoculated with 40 to 80 g grain spawn.

In order to enhance ventilation, it is preferred in the context of the present invention to close the polypropylene bags inoculated with the mycelial matter of the desired fungal species by folding instead of sealing/welding. In the context of the present invention, the polypropylene bags may be folded thrice and sealed with a clamp.

**In** order to efficiently and reproducibly induce/grow fruiting bodies of a desired fungal species *Laetiporus sulphureus* with food-grade quality, it may be particularly useful to perform in vitro cultivation described herein based on a combination of the methods and the substrates disclosed herein.

### Fruiting Bodies

In a fourth aspect, the present invention relates to a fruiting body obtained by or obtainable by performing the methods, i.e., the induction and/or growth methods, disclosed herein. In line with the methods provided herein, a fruiting body obtained by or obtainable by the methods is a fruiting body of a fungus from the genus *Laetiporus,* most preferably from fungal species *Laetiporus sulphureus.*

In a fifth aspect, the present invention relates to a fruiting body, preferably a fruiting body of a fungus from the genus *Laetiporus,* most preferably from fungal species *Laetiporus sulphureus.*

In the context of the present invention, "fruiting body" preferably refers to the mature/fully differentiated stage of mushroom. The skilled person will understand that in the context of the present invention, fruiting bodies are usually the fleshy consumed/consumable part of edible fungal species; the fruiting bodies typically consist of a dense/compact network of the mycelial matter of the fungus and/or are the mature/fully differentiated stage of mushroom and/or are larger in size compared to the above mentioned primordia (i.e. compared to the initial stage of fruiting body formation).Also developing, immature fruiting bodies may be consumed, as it is custom with other fungi species. Accordingly, in the context of the present invention, "fruiting body" can also refer to developing and/or immature fruiting bodies. "Developing and/or immature fruiting body" in this context refers primarily to amorphous, developing and/or immature fruiting body (also termed herein amorphous fruiting body or amorphous immature fruiting body or amorphous developing fruiting body) , e.g. fruiting bodies grown e.g. in the dark or under low light conditions (preferably < 100 lux), e.g. when performing step (ii) of the methods provided herein. "Developing and/or immature fruiting body" are usually larger in size compared to the primordia which are the initial, immature stage of the mushroom's fruiting bodies, as explained above. For example, a "developing and/or immature fruiting body" can have a diameter of above 7.5 mm and up to several cm, e.g. up to 10, 15 or 20 cm). Without deferring from the gist of invention, it is contemplated that (a sample of) "fruiting bodies" (e.g. to be harvested in accordance with the provided methods) as defined herein can, in addition, comprise primordia (in the sense of initial, immature stage of the mushroom's fruiting bodies or parts thereof). Preferably, (a sample of) "fruiting bodies" (e.g. to be harvested in accordance with the provided methods) as defined herein does not comprise primordia (in the sense of initial, immature stage of the mushroom's fruiting bodies or parts thereof).

A "fruiting body" herein preferably is a fresh weight fruiting body, i.e., a freshly harvested fruiting body (e.g. from shortly after harvest (like several minutes) to up to several weeks, particularly when stored at low temperatures (e.g., at 4 °C) for), as compared to a dry/dried fruiting body. Most preferably, the "fruiting body" is pasteurized (directly) after harvest. Such a pasteurized fruiting body can then be stored for up to several weeks, particularly when stored at low temperatures (e.g., at 4 °C).

Since the substrates according to the present invention are preferably free of toxins and/or substances rendering the fruiting bodies deleterious or inedible, fruiting bodies produced by using said substrates have food-grade quality. As such, the fruiting bodies described herein are free of off-tastes, including but not limited to tannic acid and/or toxins, such as ZnSO₄. "Free" means in particular that no detectable amounts are present and/or that the amount are minute, and especially the substances are not present in amounts that could cause a toxic reaction and/or would render the fruiting bodies deleterious or inedible (e.g., unsuitable for human consumption and/or not food grade quality).

Furthermore, since the substrates described herein also contain high-quality nutrients, fruiting bodies produced by using said substrates may have a high nutritional value, which even outperforms the nutritional value of wild growing mushrooms of the same species.

In the context of the present invention, the high nutritional value of the fruiting bodies particularly refers to a high amount of protein, preferably measured/indicated per gram of dry weight (DW) and/or per gram of wet sample/fresh weight.

The fruiting bodies described herein have/comprise at least 4 %, preferably at least 5 %, 6 %, more preferably at least 7 %, 8 %, 9 %, even more preferably at least 10 % or 11 % even more preferably at least 12 % of protein per gram of wet sample/fresh weight and preferably up to 13, 14 or 15 % or even more of protein per gram of wet sample/fresh weight.

Alternatively, and/or in addition, the fruiting bodies described herein have/comprise above 21 %, 22, %, 23 %, 24 %, more preferably above 25 %, 30% 35 %, 40 %, 45%, 50% of protein per gram of dry weight and up to 55 % or 60 % protein per gram of dry weight.

The skilled person will understand that the increased protein content of the induced and subsequently, grown *Laetiporus sulphureus* fruiting bodies thought to be a consequence of using the substrates of the present invention, specifically those substrates with a high percentage of an amino acid component.

The skilled person is in the position to determine the protein content of a fruiting body and any means suitable for determining the protein content known in the art may be employed. For instance, useful methods for measuring protein content in the context of the present invention, include, but are not limited to, the Kjeldahl method, Dumas method, direct measurement methods using UV-spectroscopy and refractive index measurement, Near-Infrared Reflectance and the like.

In a preferred embodiment of the present invention, the protein content of the fruiting bodies is determined by using the Dumas method e.g., by following the protocol below (see Nielsen, Nielsen (2014). Food Analysis. Springer Science & Business Media. p. 143. ISBN 9781441914774; NPL21):
The crude nitrogen content can be determined using the DUMAS method, as follows. Samples of ground mushroom (100 mg) are weighed into the special tin foil cups commonly used with high precision scales. These tin foil cups are then balled up tightly and placed into prelabelled tubes (e.g., Eppendorf Tubes^{®}) (1.5 mL). These tightly packed samples are then loaded onto an elemental analyzer (e.g., the TruSpec^{®} N series LECO, Monchengladbach, Germany). The parameters set for the run consisted of an oven temperature at 950 °C, the post burn temperature at 850 °C and the thermo electric cooler temperature at 5 °C.

The essence of this method is to use a mixture of heat and gas to initially combust the sample with the use of oxygen, then completely reduce nitrogen oxides to nitrogen and then finally filter and trap all elements beside nitrogen which is then detected and determined by the machine. The crude nitrogen content is then converted to protein % by using the well-known conversion factor 6.25 (NPL18).

### Cited Non patent literature

[NPL1] Ríos, J.-L., Andújar, I., Recio, M.-C., & Giner, R.-M. (2012). Lanostanoids from Fungi: A Group of Potential Anticancer Compounds. Journal of Natural Products, 75(11), 2016-2044. https://doi.org/10.1021/np300412h
[NPL2] Sulkowska-Ziaja, K., Muszynska, B., Gawalska, A., & Salaciak, K. (2018). Laetiporus sulphureus - CHEMICAL COMPOSITION AND MEDICINAL VALUE. Acta Scientiarum Polonorum Hortorum Cultus, 17(1), 87-96. https://doi.org/10.24326/asphc.2018.1.8
[NPL3] Grienke, U., Zoll, M., Peintner, U., & Rollinger, J. M. (2014). European medicinal polypores-A modern view on traditional uses. Journal of Ethnopharmacology, 154, 564-583.
[NPL4] Khatua, S., Ghosh, S., Acharya, K., & Acharya, K. (2017). Laetiporus sulphureus (Bull.: Fr.) Murr. as Food as Medicine. Pharmacognosy Journal, 9(6s), s1-s15. https://doi.org/10.5530/pj.2017.6s.151
[NPL5] Olennikov, D. N., Tankhaeva, L. M., & Agafonova, S. V. (2011). Antioxidant components of Laetiporus sulphureus (Bull.: Fr.) Murr. fruit bodies. Applied Biochemistry and Microbiology, 47(4), 419-425. https://doi.org/10.1134/S0003683811040107
[NPL6] Wiater, A., Pleszczynska, M., Szczodrak, J., & Janusz, G. (2012). Comparative Studies on the Induction of Trichoderma harzianum Mutanase by α-(1→3)-Glucan-Rich Fruiting Bodies and Mycelia of Laetiporus sulphureus. International Journal of Molecular Sciences, 13(8), 9584-9598. https://doi.org/10.3390/ijms13089584
[NPL7] Agafonova, S. V., Olennikov, D. N., Borovskii, G. B., & Penzina, T. A. (2007). Chemical composition of fruiting bodies from two strains of Laetiporus sulphureus. Chemistry of Natural Compounds, 43(6), 687-688.
[NPL8] Ota, Y., Hattori, T., Banik, M. T., Hagedorn, G., Sotome, K., Tokuda, S., & Abe, Y. (2009). The genus Laetiporus (Basidiomycota, Polyporales) in East Asia. Mycological Research, 113(11), 1283-1300. https://doi.org/10.1016/j.mycres.2009.08.014
[NPL9] Petrović, J., Glamoclija, J., Stojković, D. S., Ciric, A., Nikolić, M., Bukvicki, D., Guerzoni, M. E., & Sokovic, M. D. (2013). Laetiporus sulphureus, edible mushroom from Serbia: Investigation on volatile compounds, in vitro antimicrobial activity and in situ control of Aspergillus flavus in tomato paste. Food and Chemical Toxicology, 59, 297-302. https://doi.org/10.1016/j.fct.2013.06.021
[NPL10] Rapior, S., Konska, G., Guillot, J., Andary, C., & Bessiere, J.-M. (2000). Volatile composition of Laetiporus sulphureus. Cryptogamie Mycologie, 21(1), 67-72. https://doi.org/10.1016/S0181-1584(00)00109-3
[NPL11] Klaus, A., Kozarski, M., Niksic, M., Jakovljevic, D., Todorovic, N., Stefanoska, I., & Griensven, L. J. L. D. V. (2013). The edible mushroom Laetiporus sulphureus as potential source of natural antioxidants. International Journal of Food Sciences and Nutrition, 64(5), 599-610. https://doi.org/10.3109/09637486.2012.759190
[NPL12] Upadhyaya, J. (2018). Analysis of Nutritional and Nutraceutical Properties of Wild-Grown Mushrooms of Nepal. s-0038-1644943. https://doi.org/10.1055/s-0038-1644943 [NPL13] Lindner, D. L., & Banik, M. T. (2008). Molecular phylogeny of Laetiporus and other brown rot polypore genera in North America. Mycologia, 100(3), 417-430.
[NPL14] Olennikov, D. N., Agafonova, S. V., Borovskii, G. B., Penzina, T. A., & Rokhin, A. V. (2009). Water-soluble endopolysaccharides from the fruiting bodies of Laetiporus sulphureus (Bull.: Fr.) Murr. Applied biochemistry and microbiology, 45(5), 536-543.
[NPL15] Olennikov, D. N., Agafonova, S. V., Nazarova, A. V., Borovskii, G. B., & Penzina, T. A. (2008). Organic acids and carbohydrates from Laetiporus sulphureus fruiting bodies. Chemistry of Natural Compounds, 44(6), 762-763. https://doi.org/10.1007/s10600-009-9180-x
[NPL16] Pleszczynska, M., Wiater, A., Siwulski, M., & Szczodrak, J. (2013). Successful large-scale production of fruiting bodies of Laetiporus sulphureus (Bull.: Fr.) Murrill on an artificial substrate. WorldJournal of Microbiology and Biotechnology, 29(4), 753-758.
[NPL17] What Is Vegetarian Chicken and How Is It Made? (2019, July 28). Food Crumbles. https://foodcrumbles.com/vegetarian-chicken-made/
[NPL18] Rhee, K. C. (2001). Determination of total nitrogen. Current Protocols in Food Analytical Chemistry, (1), B1-2.
[NPL19] Kovács, D., & Vetter, J. (2015). Chemical composition of the mushroom Laetiporus sulphureus (Bull.) Murill., Acta Alimentaria, 44(1), 104-110.
[NPL20] Mattila, P., Salo-Väänänen, P., Könkö, K., Aro, H., & Jalava, T. (2002). Basic composition and amino acid contents of mushrooms cultivated in Finland. Journal of Agricultural and Food Chemistry, 50(22), 6419-6422.
[NPL21] Nielsen, Nielsen (2014). Food Analysis. Springer Science & Business Media. p. 143. ISBN 9781441914774.

All patents and non-patent documents cited herein are hereby incorporated by reference in their entirety.

As used herein, the terms "comprising", "including", "having" or grammatical variants thereof are to be taken as specifying the stated features, integers, steps or components but do not preclude the addition of one or more additional features, integers, steps, components or groups thereof. The terms "comprising"/"including"/"having" encompass the terms "consisting of' and "consisting essentially of'. Thus, whenever the terms "comprising"/"including"/"having" are used herein, they can be replaced by "consisting essentially of' or, preferably, by "consisting of'.

The terms "comprising"/"including"/"having" mean that any further component (or likewise features, integers, steps and the like) can be present.

The term "consisting of' means that no further component (or likewise features, integers, steps and the like) can be present.

The term "consisting essentially of' or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed product, composition, device or method and the like.

Thus, the term "consisting essentially of' means that specific further components (or likewise features, integers, steps and the like) can be present, namely those not materially affecting the essential characteristics of the product, composition, device or method. In other words, the term "consisting essentially of" (which can be interchangeably used herein with the term "comprising substantially"), allows the presence of other components in the product, composition, device or method in addition to the mandatory components (or likewise features, integers, steps and the like), provided that the essential characteristics of the product, composition, device or method are not materially affected by the presence of other components.

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts.

As used herein, the term "isolated" refers to a composition that has been removed from its in vivo location. Preferably the isolated compositions or compounds of the present invention are substantially free from other substances (e.g., other proteins or other compounds) that are present in their in-vivo location (i.e., purified or semi-purified compositions or compounds.)

As used herein the term "about" refers to ± 10%, preferably ± 5%, more preferably ± 1%.

As used herein, "a" or "an" may mean one or more. It is envisaged that concrete values disclosed herein (e.g. X g (like 10 g) or X % (like 10 %) are also disclosed as "about X" (like about X g (like about 10 g) or about X % (like about 10 %). However, it is preferred that when concrete values are disclosed herein, these concrete values are mean, i.e. without "about".

In the following, the invention is further illustrated in non-limiting examples.

### Examples

### Example 1: Impact of different CO₂ concentrations on the induction and subsequent growth of fruiting bodies of the fungus Laetiporus sulphureus.

Mycelium from collected fruiting bodies of *Laetiporus sulphureus* was isolated by incubation of fungal matter from the inner part of the fruiting body on sterile medium in petri dishes. To produce grain spawn, wheat berries where steamed until soft and mixed with 5 % (dry sample) gypsum, then autoclaved at 121 °C for 60 min. After cooling, the mixture was inoculated with mycelium and incubated for two weeks.

Polypropylene bags containing sterilized substrate inoculated with *Laetiporus sulphureus* grain spawn were transferred to five different incubation chambers. The used substrate was the same as described in Example 3 below. The bags were incubated in the dark or in very low light conditions below 100 lux at 26 °C (corresponding to first predetermined temperature) and 50 % humidity (corresponding to first predetermined humidity) with moderate ventilation, i.e., corresponding to CO₂ concentration at 5000 ppm for up to 3 weeks (corresponding to first predetermined CO₂ concentration). The CO₂ concentration increased from atmospheric levels during cultivation until it reached about 5000 ppm after 7 to 14 days of culture. The light conditions (below 100 lux), temperature (26 °C) and humidity (50 %) were maintained throughout this experiment, i.e., until primordia formed. When the bags were fully colonized, i.e., all substrate was covered in mycelium, the CO₂ concentration in the incubators which peaked at 5000 ppm (see above) was differentially decreased by ventilation as shown in **Table 1** below for Incubators 2 to 4 (corresponding to second predetermined CO₂ concentration). The CO₂ concentration in Incubator 1 was not actively regulated by ventilation, but gradually decreased due to the declining metabolization of the fungi. Induction potential of the different CO₂ concentrations was visually evaluated based on the appearance of primordia (i.e., the initial stage of fruiting body formation) (see **Table 1,** last column), which emerge from the filter patches and/or folds in the bags (see **Figure 2A****).**

**Table 1: Overview of the CO₂ concentrations tested with respect to fruiting body induction potential.**

| Fruiting body induction was visually confirmed by emergence of primordia (initial stage of fruiting bodies). Percentage of induction efficiency was calculated based on the ratio of samples (i.e., inoculated polypropylene bags) with emerging primordia (initial stage of fruiting bodies) and the total sample size. Values were then normalized to the conditions in Incubator (Inc.) 1, i.e., under conditions where the second CO₂ concentration was not actively regulated via ventilation. | | | | | | | |
|---|---|---|---|---|---|---|---|
| **Inc.** | **CO₂ concentration for induction** | **Sample size** | **Fruiting bodies emerged** | | **Induction efficiency** | **Value for induction efficiency (normalized to Inc. 1)** | **Value for induction efficiency in % (normalized to Inc. 1)** |
| | | | **Yes** | **No** | | | |
| 1 | > 2500 ppm | 162 | 23 | 139 | 14.2 % | 1 | 100% |
| 2 | > 1500 ppm | 140 | 28 | 112 | 20.0 % | 1.41 | 141% |
| 3 | > 700 ppm | 91 | 21 | 70 | 23.0 % | 1.62 | 162% |
| 4 | < 700 ppm | 53 | 40 | 13 | 75.5 % | 5.317 | 531.7% |

The percentage of induction efficiency was increased even up to 95.2 % (induction efficiency) (672.5 % compared to "Incubator 1" (high CO₂ concentrations maintained)) in a further independent experiment under conditions corresponding to the condition in "Incubator 4".

After fruiting body induction (primordia formation), temperature was decreased from 26 °C to 18 °C, humidity was increased from 50 % to 90 % and the light conditions were changed to a 14 h light regime with 500 to 3000 lux until fleshly fruiting bodies developed from the primordia (see **Figure 2B****,** **C).**

The following examples **(Examples 2 to 16)** describe the use of the fruiting body induction and growing method according to the present invention using various substrates in order to obtain fruiting bodies of a well cultivable fungus *Laetiporus sulphureus* strain. **Example 2** is based on a substrate disclosed in the prior art PL 219753. **Examples 3** to **16** are based on substrates provided herein.

### Example 2: Induction of fruiting bodies of the fungus Laetiporus sulphureus triggered by a decrease in the CO₂ concentration during in vitro culture with a substrate disclosed in the prior art.

To begin with, it is pointed out the present inventors failed to reproduce, i.e., induce and produce fruiting bodies by using the method, i.e., fruiting body induction via a decrease in temperature and/or the injection of cooled water, in combination with the substrate disclosed in PL 219753, despite attempts using several hundreds of bags, many of which got contaminated after cold water injection (see **Figure 3****).**

However, when combining the substrate disclosed in PL 219753 with the methods for inducing and growing fruiting bodies according to the present invention, it was possible to induce and grow fruiting bodies of *Laetiporus sulphureus,* at least to a certain extent.

1100 g triticale berries, 4000 g deciduous saw dust, 5000 g oak wood chips were mixed and soaked in 18000 g water for 12-16 h. 1150 g corn flour, 1150 g millet 600 g gypsum, 264 g Dolomite, 180 g chalk, 7.2 g (NH₄)₂SO₄, 7.2 g K₂HPO₄, 0.72 g MgSO₄, 180 g sucrose as well as 400 g buckwheat bran, 1500 g rye bran and 2700 g wheat bran were mixed dry and added to the soaked components. Everything was then mixed together until all ingredients were blended well. Polypropylene bags with a 5 µm filter batch were filled with 3 kg of the substrate. The top of the bags was folded thrice and fixated with a clamp. The bags were sterilized using steam at about 98°C for 3 to 4 h. When chilled to a maximum of 28 °C, the bags were transferred to a laminar flow bench. Each bag was opened and about 40 to 80 g grain spawn was added. The bags were then either folded and clamped again or sealed, and the substrate was thoroughly mixed with the grain spawn by kneading and shaking the bags. The bags were transferred to the incubation chamber, put on wire racks with a distance of about 15 cm between the bags and were incubated in the dark or in very low light conditions below 100 lux at 26 °C (corresponding to first predetermined temperature) and 50 % humidity (corresponding to first predetermined humidity) with moderate ventilation, i.e., CO₂ at 5000 ppm (corresponding to first predetermined CO₂ concentration) for up to 3 weeks. When the bags were fully colonized, i.e., all substrate was covered in mycelium, the conditions were changed to high ventilation (CO2 < 700 ppm) (corresponding to second predetermined CO₂ concentration), 18 °C (corresponding to second predetermined temperature) and 90 % humidity (corresponding to second predetermined humidity) under a 14 h light regime with 500 to 3000 lux. The (amorphous) fruiting bodies emerged from the filter patches and/or holes in the bags (see **Figure 4A****).** The fruiting efficiency is shown in **Table 2,** 2^{nd} row.

### Example 3: Induction of fruiting bodies of the fungus Laetiporus sulphureus triggered by a decrease in the CO₂ concentration during in vitro culture with a substrate provided herein.

The protocol of Example 3 is identical to the protocol of Example 1 (Incubator 4) with the exception that simultaneously with the change to high ventilation (CO₂ < 700 ppm), temperature was decreased from 26 °C to 18 °C, humidity was increased from 50 % to 90 % and the light conditions were changed to a 14 h light regime with 500 to 3000 lux. The induction efficiency was comparable with that of Example 1 (Incubator 4). The experiment shows that the induction efficiency (induction of fruiting body/primordia) is increased by the incubation at a second predetermined CO₂ concentration. It is thought that temperature decrease, increase in humidity and increase in light have no effect on inducation efficiency; however, they increase the quality of the formed fruiting bodies (e.g., at higher temperatures mycelium is less dense).

2000 g wheat berries, 1800 g beech saw dust, 8200 g beech wood chips (between 5 and 20 mm) and 1200 g hemp fiber are mixed and soaked in 16000 g water for 12-16 h. 5000 g wheat bran, 1300 g corn flour, 670 g gypsum, 300 g Dolomite, 200 g CaCO₃, 200 g dextrose and 500 g lin meal are mixed dry and added to the soaked wood chips. Everything is then mixed together until all ingredients are blended well. Polypropylene bags with a 5 µm filter batch are filled with 3 kg of the substrate each. The top of the bags is folded thrice and fixated with a clamp. The bags are sterilized using steam at about 98°C for 3 to 4 h. When chilled to a maximum of 28 °C, the bags are transferred to a laminar flow bench. Each bag is opened and about 40 to 80 g grain spawn is added. The bags are then either folded and clamped again or sealed, and the substrate is thoroughly mixed with the grain spawn by kneading and shaking the bags. The bags are transferred to the incubation chamber, put on wire racks with a distance of about 15 cm between the bags and are incubated in the dark or in very low light conditions below 100 lux at 26 °C and 50 % humidity with moderate ventilation, i.e., CO₂ at 5000 ppm for up to 3 weeks. When the bags are fully colonized, i.e., all substrate is covered in mycelium, the conditions are changed to high ventilation (CO2 < 700 ppm), 18 °C and 90 % humidity under a 14 h light regime with 500 to 3000 lux. The fruiting bodies emerge from the filter patches and/or folds in the bags (see **Figure 4B****).** The fruiting bodies are ready to harvest after about 7 to 10 days. The fruiting efficiency is shown in **Table 2,** 3^{rd} row.

### Example 4: Induction of fruiting bodies of the fungus Laetiporus sulphureus triggered by a decrease in the CO₂ concentration during in vitro culture in combination with a substrate provided herein.

The protocol of Example 4 is identical to the protocol of Example 3 with the exception that a different substrate was used.

2000 g wheat berries, 1800 g beech saw dust, 8200 g beech wood chips (between 5 and 20 mm) and 1200 g hemp fiber are mixed and soaked in 11500 g water for 12-16 h. 5000 g wheat bran, 1300 g corn flour, 670 g gypsum, 300 g Dolomite, 200 g CaCO₃, 200 g dextrose and 500 g lin meal are mixed dry and added to the soaked wood chips. Everything is then mixed together until all ingredients are blended well. Polypropylene bags with a 5 µm filter batch are filled with 3 kg of the substrate each. The top of the bags is folded thrice and fixated with a clamp. The bags are sterilized using steam at about 98°C for 3 to 4 h. When chilled to a maximum of 28 °C, the bags are transferred to a laminar flow bench. Each bag is opened and about 40 to 80 g grain spawn is added. The bags are then either folded and clamped again or sealed, and the substrate is thoroughly mixed with the grain spawn by kneading and shaking the bags. The bags are transferred to the incubation chamber, put on wire racks with a distance of about 15 cm between the bags and are incubated in the dark or in very low light conditions below 100 lux at 26 °C and 50 % humidity with moderate ventilation, i.e., CO₂ at 5000 ppm for up to 3 weeks. When the bags are fully colonized, i.e., all substrate is covered in mycelium, the conditions are changed to high ventilation (CO2 < 700 ppm), 18 °C and 90 % humidity under a 14 h light regime with 500 to 3000 lux. The fruiting efficiency is shown in **Table 2,** 4^{th} row.

### Example 5: Induction of fruiting bodies of the fungus Laetiporus sulphureus triggered by a decrease in the CO₂ concentration during in vitro culture in combination with a substrate provided herein.

The protocol of Example 5 is identical to the protocol of Example 3 with the exception that a different substrate was used.

2000 g wheat berries, 1800 g beech saw dust, 8200 g beech wood chips (between 5 and 20 mm) and 1200 g hemp fiber are mixed and soaked in 2600 g water for 12-16 h. 5000 g wheat bran, 1300 g corn flour, 670 g gypsum, 300 g Dolomite, 200 g CaCO₃, 200 g dextrose and 500 g lin meal are mixed dry and added to the soaked wood chips. Everything is then mixed together until all ingredients are blended well. Polypropylene bags with a 5 µm filter batch are filled with 3 kg of the substrate each. The top of the bags is folded thrice and fixated with a clamp. The bags are sterilized using steam at about 98°C for 3 to 4 h. When chilled to a maximum of 28 °C, the bags are transferred to a laminar flow bench. Each bag is opened and about 40 to 80 g grain spawn is added. The bags are then either folded and clamped again or sealed, and the substrate is thoroughly mixed with the grain spawn by kneading and shaking the bags. The bags are transferred to the incubation chamber, put on wire racks with a distance of about 15 cm between the bags and are incubated in the dark or in very low light conditions below 100 lux at 26 °C and 50 % humidity with moderate ventilation, i.e., CO₂ at 5000 ppm for up to 3 weeks. When the bags are fully colonized, i.e., all substrate is covered in mycelium, the conditions are changed to high ventilation (CO2 < 700 ppm), 18 °C and 90 % humidity under a 14 h light regime with 500 to 3000 lux. The fruiting efficiency is shown in **Table 2,** 5^{th} row.

### Example 6: Induction of fruiting bodies of the fungus Laetiporus sulphureus triggered by a decrease in the CO₂ concentration during in vitro culture in combination with a substrate provided herein.

The protocol of Example 6 is identical to the protocol of Example 3 with the exception that a different substrate was used.

2000 g wheat berries, 1800 g beech saw dust, 8200 g beech wood chips (between 5 and 20 mm) and 1200 g hemp fiber are mixed and soaked in 32500 g water for 12-16 h. 5000 g wheat bran, 1300 g corn flour, 670 g gypsum, 300 g Dolomite, 200 g CaCO₃, 200 g dextrose and 500 g lin meal are mixed dry and added to the soaked wood chips. Everything is then mixed together until all ingredients are blended well. Polypropylene bags with a 5 µm filter batch are filled with 3 kg of the substrate each. The top of the bags is folded thrice and fixated with a clamp. The bags are sterilized using steam at about 98°C for 3 to 4 h. When chilled to a maximum of 28 °C, the bags are transferred to a laminar flow bench. Each bag is opened and about 40 to 80 g grain spawn is added. The bags are then either folded and clamped again or sealed, and the substrate is thoroughly mixed with the grain spawn by kneading and shaking the bags. The bags are transferred to the incubation chamber, put on wire racks with a distance of about 15 cm between the bags and are incubated in the dark or in very low light conditions below 100 lux at 26 °C and 50 % humidity with moderate ventilation, i.e., CO₂ at 5000 ppm for up to 3 weeks.

When the bags are fully colonized, i.e., all substrate is covered in mycelium, the conditions are changed to high ventilation (CO2 < 700 ppm), 18 °C and 90 % humidity under a 14 h light regime with 500 to 3000 lux. The fruiting efficiency is shown in **Table 2,** 6^{th} row.

### Example 7: Induction of fruiting bodies of the fungus Laetiporus sulphureus triggered by a decrease in the CO₂ concentration during in vitro culture in combination with a substrate provided herein.

The protocol of Example 7 is identical to the protocol of Example 3 with the exception that a different substrate was used.

2000 g wheat berries, 1800 g beech saw dust, 8200 g beech wood chips (sized between 5 and 20 mm) and 1200 g hemp fiber were mixed and soaked in 16000 g water for 12-16 h. 1300 g corn flour, 670 g gypsum, 300 g Dolomite, 200 g CaCO₃, 200 g dextrose as well as 500 g lin meal, 1000 g sunflower meal, 1000 g poppy seed meal, 1000 g hemp meal, 1000 g pumpkin seed meal and 1000 g rape meal were mixed dry and added to the soaked components. Everything was then mixed together until all ingredients were blended well. Polypropylene bags with a 5 µm filter batch were filled with 3 kg of the substrate. The top of the bags was folded thrice and fixated with a clamp. The bags were sterilized using steam at about 98°C for 3 to 4 h. When chilled to a maximum of 28 °C, the bags are transferred to a laminar flow bench. Each bag was opened and about 40 to 80 g grain spawn was added. The bags were then either folded and clamped again or sealed, and the substrate was thoroughly mixed with the grain spawn by kneading and shaking the bags. The bags were transferred to the incubation chamber, put on wire racks with a distance of about 15 cm between the bags and were incubated in the dark or in very low light conditions below 100 lux at 26 °C and 50 % humidity with moderate ventilation, i.e., CO₂ at 5000 ppm for up to 3 weeks. When the bags were fully colonized, i.e., all substrate was covered in mycelium, the conditions were changed to high ventilation (CO2 < 700 ppm), 18 °C and 90 % humidity under a 14 h light regime with 500 to 3000 lux. The fruiting bodies emerged from the filter patches and/or holes in the bags. The fruiting efficiency is shown in **Table 2,** 7^{th} row.

### Example 8: Induction of fruiting bodies of the fungus Laetiporus sulphureus triggered by a decrease in the CO₂ concentration during in vitro culture in combination with a substrate provided herein.

The protocol of Example 8 is identical to the protocol of Example 3 with the exception that a different substrate was used.

2000 g wheat berries, 1800 g beech saw dust, 8200 g beech wood chips (sized between 5 and 20 mm) and 1200 g hemp fiber were mixed and soaked in 16000 g water for 12-16 h. 1300 g corn flour, 670 g gypsum, 300 g Dolomite, 200 g CaCO₃, 200 g dextrose as well as 500 g lin meal and 5000 g sunflower meal were mixed dry and added to the soaked components. Everything was then mixed together until all ingredients were blended well. Polypropylene bags with a 5 µm filter batch were filled with 3 kg of the substrate. The top of the bags was folded thrice and fixated with a clamp. The bags were sterilized using steam at about 98 °C for 3 to 4 h. When chilled to a maximum of 28 °C, the bags were transferred to a laminar flow bench. Each bag was opened and about 40 to 80 g grain spawn was added. The bags were then either folded and clamped again or sealed, and the substrate is thoroughly mixed with the grain spawn by kneading and shaking the bags. The bags were transferred to the incubation chamber, put on wire racks with a distance of about 15 cm between the bags and were incubated in the dark or in very low light conditions below 100 lux at 26 °C and 50 % humidity with moderate ventilation, i.e., CO₂ at 5000 ppm for up to 3 weeks. When the bags were fully colonized, i.e., all substrate was covered in mycelium, the conditions were changed to high ventilation (CO2 < 700 ppm), 18 °C and 90 % humidity under a 14 h light regime with 500 to 3000 lux. The fruiting bodies emerged from the filter patches and/or holes in the bags. The fruiting efficiency is shown in **Table 2,** 8^{th} row.

### Example 9: Induction of fruiting bodies of the fungus Laetiporus sulphureus triggered by a decrease in the CO₂ concentration during in vitro culture in combination with a substrate provided herein.

The protocol of Example 9 is identical to the protocol of Example 3 with the exception that a different substrate was used.

2000 g wheat berries, 1800 g beech saw dust, 8200 g beech wood chips (sized between 5 and 20 mm) and 1200 g hemp fiber were mixed and soaked in 16000 g water for 12-16 h. 1300 g corn flour, 670 g gypsum, 300 g Dolomite, 200 g CaCO₃, 200 g dextrose as well as 500 g lin meal and 5000 g beer yeast were mixed dry and added to the soaked components. Everything was then mixed together until all ingredients were blended well. Polypropylene bags with a 5 µm filter batch were filled with 3 kg of the substrate. The top of the bags was folded thrice and fixated with a clamp. The bags were sterilized using steam at about 98 °C for 3 to 4 h. When chilled to a maximum of 28 °C, the bags were transferred to a laminar flow bench. Each bag was opened and about 40 to 80 g grain spawn was added. The bags were then either folded and clamped again or sealed, and the substrate was thoroughly mixed with the grain spawn by kneading and shaking the bags. The bags were transferred to the incubation chamber, put on wire racks with a distance of about 15 cm between the bags and were incubated in the dark or in very low light conditions below 100 lux at 26 °C and 50 % humidity with moderate ventilation, i.e., CO₂ at 5000 ppm for up to 3 weeks. When the bags were fully colonized, i.e., all substrate was covered in mycelium, the conditions were changed to high ventilation (CO2 < 700 ppm), 18 °C and 90 % humidity under a 14 h light regime with 500 to 3000 lux. The fruiting bodies emerged from the filter patches and/or holes in the bags. The fruiting efficiency is shown in **Table 2,** 9^{th} row.

### Example 10: Induction of fruiting bodies of the fungus Laetiporus sulphureus triggered by a decrease in the CO₂ concentration during in vitro culture in combination with a substrate provided herein.

The protocol of Example 10 is identical to the protocol of Example 3 with the exception that a different substrate was used.

2000 g wheat berries, 1800 g beech saw dust, 4200 g beech wood chips (sized between 5 and 20 mm) as well as 1200 g hemp fiber, 2000 g lin fiber and 2000 g cut straw were mixed and soaked in 16000 g water for 12-16 h. 5000 g wheat bran, 1300 g corn flour, 670 g gypsum, 300 g Dolomite, 200 g CaCO₃, 200 g dextrose and 500 g lin meal were mixed dry and added to the soaked components. Everything was then mixed together until all ingredients were blended well. 3 Polypropylene bags with a 5 µm filter batch were filled with 3 kg of the substrate. The top of the bags was folded thrice and fixated with a clamp. The bags were sterilized using steam at about 98 °C for 3 to 4 h. When chilled to a maximum of 28 °C, the bags were transferred to a laminar flow bench. Each bag was opened and about 40 to 80 g grain spawn was added. The bags were then either folded and clamped again or sealed, and the substrate was thoroughly mixed with the grain spawn by kneading and shaking the bags. The bags were transferred to the incubation chamber, put on wire racks with a distance of about 15 cm between the bags and were incubated in the dark or in very low light conditions below 100 lux at 26 °C and 50 % humidity with moderate ventilation, i.e., CO₂ at 5000 ppm for up to 3 weeks. When the bags were fully colonized, i.e., all substrate is covered in mycelium, the conditions were changed to high ventilation (CO2 < 700 ppm), 18 °C and 90 % humidity under a 14 h light regime with 500 to 3000 lux. The fruiting bodies emerged from the filter patches and/or holes in the bags. The fruiting efficiency is shown in **Table 2,** 10^{th} row.

### Example 11: Induction of fruiting bodies of the fungus Laetiporus sulphureus triggered by a decrease in the CO₂ concentration during in vitro culture in combination with a substrate provided herein.

The protocol of Example 11 is identical to the protocol of Example 3 with the exception that a different substrate was used.

3000 g beech saw dust, 12000 g beech wood chips (sized between 5 and 20 mm) as well as 3000 g hemp fiber were mixed and soaked in 16000 g water for 12-16 h. 1300 g wheat bran, 670 g gypsum, 300 g Dolomite, 200 g CaCO₃ and 500 g lin meal were mixed dry and added to the soaked components. Everything was then mixed together until all ingredients were blended well. Polypropylene bags with a 5 µm filter batch were filled with 3 kg of the substrate. The top of the bags was folded thrice and fixated with a clamp. The bags were sterilized using steam at about 98 °C for 3 to 4 h. When chilled to a maximum of 28 °C, the bags were transferred to a laminar flow bench. Each bag was opened and about 40 to 80 g grain spawn was added. The bags were then either folded and clamped again or sealed, and the substrate was thoroughly mixed with the grain spawn by kneading and shaking the bags. The bags were transferred to the incubation chamber, put on wire racks with a distance of about 15 cm between the bags and were incubated in the dark or in very low light conditions below 100 lux at 26 °C and 50 % humidity with moderate ventilation, i.e., CO₂ at 5000 ppm for up to 3 weeks. When the bags were fully colonized, i.e., all substrate was covered in mycelium, the conditions were changed to high ventilation (CO2 < 700 ppm), 18 °C and 90 % humidity under a 14 h light regime with 500 to 3000 lux. The fruiting bodies emerged from the filter patches and/or holes in the bags. The fruiting efficiency is shown in **Table 2,** 11^{th} row.

### Example 12: Induction of fruiting bodies of the fungus Laetiporus sulphureus triggered by a decrease in the CO₂ concentration during in vitro culture with a substrate provided herein.

The protocol of Example 12 is identical to the protocol of Example 3 with the exception that a different substrate was used.

2000 g wheat berries, 1800 g beech saw dust, 8200 g beech wood chips (sized between 5 and 20 mm) as well as 1200 g hemp fiber were mixed and soaked in 18000 g water for 12-16 h. 5000 g wheat bran, 1300 g corn flour, 670 g gypsum, 300 g Dolomite, 200 g CaCO₃, 200 g dextrose and 3000 g lin meal (deoiled) were mixed dry and added to the soaked components. Everything was then mixed together until all ingredients were blended well. 3 Polypropylene bags with a 5 µm filter batch were filled with 3 kg of the substrate. The top of the bags was folded thrice and fixated with a clamp. The bags were sterilized using steam at about 98°C for 3 to 4 h. When chilled to a maximum of 28 °C, the bags were transferred to a laminar flow bench. Each bag was opened and about 40 to 80 g grain spawn was added. The bags were then either folded and clamped again or sealed, and the substrate was thoroughly mixed with the grain spawn by kneading and shaking the bags. The bags were transferred to the incubation chamber, put on wire racks with a distance of about 15 cm between the bags and were incubated in the dark or in very low light conditions below 100 lux at 26 °C and 50 % humidity with moderate ventilation, i.e., CO₂ at 5000 ppm for up to 3 weeks. When the bags were fully colonized, i.e., all substrate is covered in mycelium, the conditions were changed to high ventilation (CO2 < 700 ppm), 18 °C and 90 % humidity under a 14 h light regime with 500 to 3000 lux. The fruiting bodies emerged from the filter patches and/or holes in the bags. The fruiting efficiency is shown in **Table 2,** 12^{th} row.

### Example 13: Induction of fruiting bodies of the fungus Laetiporus sulphureus triggered by a decrease in the CO₂ concentration during in vitro culture with a substrate provided herein.

The protocol of Example 13 is identical to the protocol of Example 3 with the exception that a different substrate was used.

2000 g wheat berries, 1800 g beech saw dust and 9400 g beech wood chips (sized between 5 and 20 mm) were mixed and soaked in 16000 g water for 12-16 h. 5000 g wheat bran, 1300 g corn flour, 670 g gypsum, 300 g Dolomite, 200 g CaCO₃, 200 g dextrose and 500 g lin meal (deoiled) were mixed dry and added to the soaked components. Everything was then mixed together until all ingredients were blended well. 3 Polypropylene bags with a 5 µm filter batch were filled with 3 kg of the substrate. The top of the bags was folded thrice and fixated with a clamp. The bags were sterilized using steam at about 98°C for 3 to 4 h. When chilled to a maximum of 28 °C, the bags were transferred to a laminar flow bench. Each bag was opened and about 40 to 80 g grain spawn was added. The bags were then either folded and clamped again or sealed, and the substrate was thoroughly mixed with the grain spawn by kneading and shaking the bags. The bags were transferred to the incubation chamber, put on wire racks with a distance of about 15 cm between the bags and were incubated in the dark or in very low light conditions below 100 lux at 26 °C and 50 % humidity with moderate ventilation, i.e., CO₂ at 5000 ppm for up to 3 weeks. When the bags were fully colonized, i.e., all substrate is covered in mycelium, the conditions were changed to high ventilation (CO2 < 700 ppm), 18 °C and 90 % humidity under a 14 h light regime with 500 to 3000 lux. The fruiting bodies emerged from the filter patches and/or holes in the bags. The fruiting efficiency is shown in **Table 2,** 13^{th} row.

### Example 14: Induction of fruiting bodies of the fungus Laetiporus sulphureus triggered by a decrease in the CO₂ concentration during in vitro culture with a substrate provided herein.

The protocol of Example 14 is identical to the protocol of Example 3 with the exception that a different substrate was used.

2000 g wheat berries, 1800 g beech saw dust, 8200 g beech wood chips (sized between 5 and 20 mm) as well as 1200 g hemp fiber were mixed and soaked in 16000 g water for 12-16 h. 5500 g rape meal, 1300 g corn flour, 670 g gypsum, 300 g Dolomite, 200 g CaCO₃ and 200 g dextrose were mixed dry and added to the soaked components. Everything was then mixed together until all ingredients were blended well. 3 Polypropylene bags with a 5 µm filter batch were filled with 3 kg of the substrate. The top of the bags was folded thrice and fixated with a clamp. The bags were sterilized using steam at about 98°C for 3 to 4 h. When chilled to a maximum of 28 °C, the bags were transferred to a laminar flow bench. Each bag was opened and about 40 to 80 g grain spawn was added. The bags were then either folded and clamped again or sealed, and the substrate was thoroughly mixed with the grain spawn by kneading and shaking the bags. The bags were transferred to the incubation chamber, put on wire racks with a distance of about 15 cm between the bags and were incubated in the dark or in very low light conditions below 100 lux at 26 °C and 50 % humidity with moderate ventilation, i.e., CO₂ at 5000 ppm for up to 3 weeks. When the bags were fully colonized, i.e., all substrate is covered in mycelium, the conditions were changed to high ventilation (CO2 < 700 ppm), 18 °C and 90 % humidity under a 14 h light regime with 500 to 3000 lux. The fruiting bodies emerged from the filter patches and/or holes in the bags. The fruiting efficiency is shown in **Table 2,** 14^{th} row.

### Example 15: Induction of fruiting bodies of the fungus Laetiporus sulphureus triggered by a decrease in the CO₂ concentration during in vitro culture with a substrate provided herein.

The protocol of Example 15 is identical to the protocol of Example 3 with the exception that a different substrate was used.

2000 g wheat berries, 1800 g beech saw dust, 8200 g beech wood chips (sized between 5 and 20 mm) as well as 1200 g hemp fiber were mixed and soaked in 16000 g water for 12-16 h. 1000 g wheat bran, 5000 g corn flour, 670 g gypsum, 300 g Dolomite, 200 g CaCO₃, 200 g dextrose and 500 g lin meal (deoiled) were mixed dry and added to the soaked components. Everything was then mixed together until all ingredients were blended well. 3 Polypropylene bags with a 5 µm filter batch were filled with 3 kg of the substrate. The top of the bags was folded thrice and fixated with a clamp. The bags were sterilized using steam at about 98°C for 3 to 4 h. When chilled to a maximum of 28 °C, the bags were transferred to a laminar flow bench. Each bag was opened and about 40 to 80 g grain spawn was added. The bags were then either folded and clamped again or sealed, and the substrate was thoroughly mixed with the grain spawn by kneading and shaking the bags. The bags were transferred to the incubation chamber, put on wire racks with a distance of about 15 cm between the bags and were incubated in the dark or in very low light conditions below 100 lux at 26 °C and 50 % humidity with moderate ventilation, i.e., CO₂ at 5000 ppm for up to 3 weeks. When the bags were fully colonized, i.e., all substrate is covered in mycelium, the conditions were changed to high ventilation (CO2 < 700 ppm), 18 °C and 90 % humidity under a 14 h light regime with 500 to 3000 lux. The fruiting bodies emerged from the filter patches and/or holes in the bags. The fruiting efficiency is shown in **Table 2,** penultimate row.

### Example 16: Induction of fruiting bodies of the fungus Laetiporus sulphureus triggered by a decrease in the CO₂ concentration during in vitro culture with a substrate provided herein.

The protocol of Example 16 is identical to the protocol of Example 3 with the exception that a different substrate was used.

2000 g wheat berries, 1800 g beech saw dust and 9400 g hemp fiber were mixed and soaked in 16000 g water for 12-16 h. 5000 g wheat bran, 1300 g corn flour, 670 g gypsum, 300 g Dolomite, 200 g CaCO₃, 200 g dextrose and 500 g lin meal (deoiled) were mixed dry and added to the soaked components. Everything was then mixed together until all ingredients were blended well. 3 Polypropylene bags with a 5 µm filter batch were filled with 3 kg of the substrate. The top of the bags was folded thrice and fixated with a clamp. The bags were sterilized using steam at about 98°C for 3 to 4 h. When chilled to a maximum of 28 °C, the bags were transferred to a laminar flow bench. Each bag was opened and about 40 to 80 g grain spawn was added. The bags were then either folded and clamped again or sealed, and the substrate was thoroughly mixed with the grain spawn by kneading and shaking the bags. The bags were transferred to the incubation chamber, put on wire racks with a distance of about 15 cm between the bags and were incubated in the dark or in very low light conditions below 100 lux at 26 °C and 50 % humidity with moderate ventilation, i.e., CO₂ at 5000 ppm for up to 3 weeks. When the bags were fully colonized, i.e., all substrate is covered in mycelium, the conditions were changed to high ventilation (CO2 < 700 ppm), 18 °C and 90 % humidity under a 14 h light regime with 500 to 3000 lux. The fruiting bodies emerged from the filter patches and/or holes in the bags. The fruiting efficiency is shown in **Table 2,** ultimate row.

**Table 2: Overview of the substrates tested with respect to fruiting body induction potential.**

| Fruiting body induction was visually confirmed by emergence of primordia (i.e., initial stage of fruiting bodies). Percentage of induction efficiency was calculated based on the ratio of samples (i.e., inoculated polypropylene bags) with emerging fruiting bodies and the total sample size. Induction efficiency was further normalized with respect to Example 2, i.e., relating to the substrate disclosed in PL 219753. | | | | | | |
|---|---|---|---|---|---|---|
| **Substrate** | **CO₂ concentration for induction** | **Sample size** | **Fruiting bodies emerged** | | **Induction efficiency** | **Value for the induction efficiency in % (normalized to Ex. 2)** |
| | | | **Yes** | **No** | | |
| Ex. 2 | < 700 ppm | 14 | 1 | 13 | 7.1% | 100% |
| Ex. 3 | < 700 ppm | 146 | 115 | 31 | 78.8 % | 1110 % |
| Ex. 4 | < 700 ppm | 12 | 10 | 2 | 16.7 % | 235 % |
| Ex. 5 | < 700 ppm | 33 | 13 | 20 | 39.4 % | 555 % |
| Ex. 6 | < 700 ppm | 18 | 6 | 12 | 33.3 % | 469 % |
| Ex. 7 | < 700 ppm | 12 | 7 | 5 | 58.3 % | 821 % |
| Ex. 8 | < 700 ppm | 12 | 4 | 8 | 33.3 % | 469 % |
| Ex. 9 | < 700 ppm | 12 | 3 | 9 | 25.0 % | 352% |
| Ex. 10 | < 700 ppm | 11 | 3 | 8 | 27.3 % | 385 % |
| Ex. 11 | < 700 ppm | 13 | 3 | 10 | 23.0 % | 324 % |
| Ex. 12 | < 700 ppm | 156 | 49 | 107 | 31.4% | 422 % |
| Ex. 13 | < 700 ppm | 14 | 2 | 12 | 14.3 % | 201 % |
| Ex. 14 | < 700 ppm | 20 | 3 | 17 | 15% | 211 % |
| Ex. 15 | < 700 ppm | 12 | 2 | 10 | 16.7 % | 235 % |
| Ex. 16 | < 700 ppm | 18 | 3 | 15 | 16.7 % | 235 % |

As shown in **Table 2,** with all substrates an induction of fruiting bodies was achieved when the herein provided method was applied, i.e., involving a decrease of the CO₂ concentration. The induction frequency was higher when the herein provided substrates were used compared to the substrate disclosed in PL 219753.

The following example **(Example 17)** characterizes the *Laetiporus sulphureus* fruiting bodies harvested after performing the present invention with respect to their biochemical composition in comparison to fruiting bodies of the same species which were picked from the wild.

### Example 17

The fact that mushrooms may adsorb ingredients from the substrate on which they grow enables to tweak the biochemical composition of the mushroom based on the substrates used for culture.

The fruiting of five different *Laetiporus sulphureus* samples from independent experiments was induced via a CO₂-decrease and fruiting bodies were grown in vitro based on the methods as used in Examples 2 to 16 and substrate according to the present invention. Specifically, the used substrate contained an amino acid component as the substrate used above in Example 6, i.e., the amino acid component amounted to approx. 41.2 % of the total dry weight of the substrate and is a mixture of wheat berries, wheat bran, corn flour and (deoiled) lin meal (amounting to 9.4 %, 23.4 %, 6.1 % and 2.3 % of the total dry weight of the substrate, respectively).

The biochemical composition of the in vitro cultivated *Laetiporus sulphureus* strains was compared to two wild *Laetiporus sulphureus* strains which were picked in Bavaria, Germany. Specifically, the fruiting bodies were analyzed with respect to moisture, ash, dry matter, protein, fat and carbohydrate content. The results are summarized in **Table 3** below. The therein shown increased protein content of the fruiting bodies obtained in accordance with the invention compared to the wild-type fruiting bodies is primarily attributed to the amino acid component of the herein used substrate.

### Fat Content

Ground up mushroom samples (~2.50 g) were placed into a centrifuge tube with a screw cap. Then 2.5 mL of HCl (8 M/25 %) and 2.5 mL of Toluene were added to the tube and shaken to ensure a full suspension of the sample. The tubes were then placed into a beaker of sand and placed into an oven (120 °C) for ~2 hours. The tubes were cooled down and then centrifuged at 2000 - 3000 rpm for 2 minutes. The top phase was aliquoted (1-2 mL) into a tared glass beaker and the solvent was evaporated using a hair dryer in a fume hood until the odor of toluene disappears. Once the beakers were cooled down, the samples were weighed. Then they were dried and weighed again until the mass stayed the same (1.0 mg deviation/difference). The fat content of the mushroom samples per dry weight (DW) is shown in **Table 3,** 3^{rd} row from the bottom.

### Protein Content

The crude nitrogen content was determined using the DUMAS method. Samples of ground mushroom (100 mg) were weighed into the special tin foil cups commonly used on the LECO instrument. These tin foil cups were then balled up tightly and placed into prelabelled Eppendorf Tubes^{®} (1.5 mL). These tightly packed samples were then loaded onto the TruSpec^{®} N series (LECO, Monchengladbach, Germany). The parameters set for the run consisted of an oven temperature at 950 °C, the post burn temperature at 850 °C and the thermo electric cooler temperature at 5 °C. The essence of this method is to use a mixture of heat and gas to initially combust the sample with the use of oxygen, then completely reduce nitrogen oxides to nitrogen and then finally filter and trap all elements beside nitrogen which is then detected and determined by the machine. The crude nitrogen content was then converted to protein % by using the well-known conversion factor 6.25 (NPL18). The protein content of the mushroom samples per fresh weight (FW) and dry weight (DW) is shown in **Table 3,** 6^{th} and 7^{th} row, respectively. Protein content determined for fresh weight (FW) can be readily converted into protein content for dry weight (DW) and vice versa.

### Carbohydrate Content

The carbohydrate content of the mushroom samples was calculated with the following formula: total carbohydrate (g/100g fresh weight (FW)) = 100 - moisture (%) - protein content (%/FW)

- crude fat (%/FW) - ash (%/FW) (NPL19, NPL20). The carbohydrate content of the mushroom samples per fresh weight (FW) and dry weight (DW) is shown in **Table 3,** penultimate and ultimate row, respectively. The terms fresh weight and wet sample are used interchangeably herein.

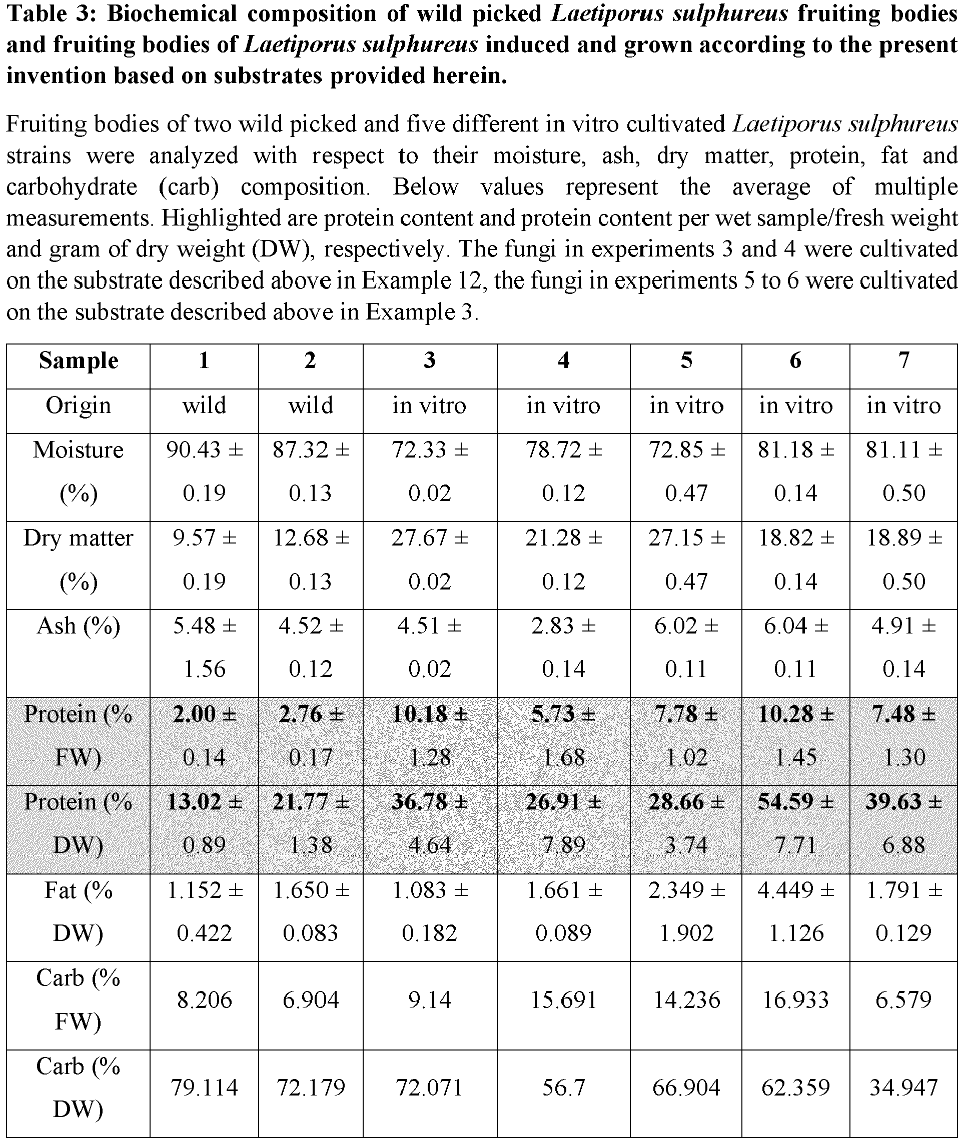

### Summary

In sum, **Example 1** shows that induction of fruiting bodies of the fungal species *Laetiporus sulphureus* can be triggered by a decrease in the CO₂ concentration from substrate colonized by mycelium (see **Table 1**). **Examples 1** and **3** furthermore show, that the second predetermined temperature, second predetermined humidity and the light regimen can be adjusted after or simultaneously with the decrease in the CO₂ concentration. **Example 2** shows that fruiting bodies of *Laetiporus sulphureus* can be induced/grown when the method of the present invention is combined with a known substrate and as such that the method of the present invention is not limited to the use of a substrate provided herein (see **Table 2**, 2^{nd} row). **Examples 3** to **16** show that combining the methods of the present invention with the substrates provided herein may further improve induction and consequently, growth of fruiting bodies of the fungal species *Laetiporus sulphureus* (see **Table 2**, 3^{rd} to last row) compared to prior art methods. No fruiting bodies were obtained, and cultures got contaminated when the method and the substrate disclosed in PL 219753 were used. When the method of the invention and substrate known from the art PL 219753 was used **(Example 2),** the formation of fruiting bodies was induced (see **Table 2**, 2^{nd} row). Furthermore, it is exemplarily shown that a variety of substrates falling under the scope of the present invention is suitable to induce/produce *Laetiporus sulphureus* fruiting bodies when used in combination with the methods of the present invention. **Examples 3 to 6** show that fruiting can be induced, and fruiting bodies grow based on different predetermined water contents of the substrates, e.g., 42.8 %, 30.8 %, 54.9 % and 60.3 %, respectively (see **Table 2,** 3^{rd} to 6^{th} row). **Example 11** shows that the presence of a saccharated component as defined herein is not absolutely necessary in the substrates used herein, although it may be advantageous, in order to induce fruiting body formation (see **Table 2,** 7^{th} row). With this respect, **Examples 7** to **10, 12** and **14** to **16** further show that fruiting can be induced, and fruiting bodies grow based on different types of the amino acid component, e.g., mixtures of grain and (different types of) meal, mixtures of grain, meal and nutritional yeast as well as mixtures of grain, meal and bran, respectively (see corresponding experiments in **Table 2). Example 9** shows that fruiting can be induced/grown in the absence of a fibrous component. **Example 17** shows that the *Laetiporus sulphureus* fruiting bodies induced and grown by using the methods/substrates of the present invention have an improved nutritional value, specifically, an increased protein content compared to wild-growing specimen (see Table 3, 6^{th} and 7^{th} row). The increased protein content of the induced and subsequently, grown *Laetiporus sulphureus* fruiting bodies is a consequence of using the methods and/or substrates of the present invention.

## Claims

1. A method for inducing fruiting body formation of a fungus from the genus *Laetiporus* comprising the steps:
a) incubating a substrate inoculated with the fungus at a first predetermined CO₂ concentration; and
b) incubating the substrate at a second predetermined CO₂ concentration,
wherein the second predetermined CO₂ concentration is lower than the first predetermined CO₂ concentration.

2. The method according to claim 1, wherein the first predetermined CO₂ concentration in step a) is above atmospheric levels, preferably above 700 ppm, more preferably above 1000 ppm, even more preferably above 1500 ppm.

3. The method according to claim 1 or 2, wherein the second predetermined CO₂ concentration is below 1500 ppm, preferably below 1000 ppm, more preferably below 700 ppm, more preferably at atmospheric levels.

4. The method according to any one of claims 1 to 3, wherein the method comprises a step of decreasing the first predetermined CO₂ concentration to a second predetermined CO₂ concentration.

5. The method according to claim 4, wherein the CO₂ concentration is decreased within 5 days, preferably within 24 hours, preferably within 12 hours, even more preferably within 3 hours.

6. A method for growing a fruiting body of a fungus from the genus *Laetiporus sulphureus* comprising the steps:
i) inducing fruiting body formation according to any one of claims 1 to 5;
ii) incubating the substrate until a fruiting body has grown;
iii) harvesting the fruiting body.

7. The method according to claim 6, wherein the incubation in step ii) is performed at the second predetermined CO₂ concentration, a second predetermined temperature and/or a second predetermined humidity.

8. The method according to claim 7, wherein the second predetermined temperature is 12 °C to 26 °C, preferably 16 °C to 22 °C, more preferably is 18 °C and/or the second predetermined humidity is above 40 %, preferably above 50 %, more preferably above 70%, even more preferably above 80 %, most preferably is 90 %.

9. The method according to any one of claims 1 to 8, wherein the substrate comprises:
a) a cellulose component;
b) an amino acid component; and
c) a mineral component;
d) and, optionally, a fibrous component.

10. A fruiting body of a fungus obtained or obtainable by the method of any one of claims 1 to 9.

11. A fruiting body of a fungus from the genus *Laetiporus*,
the fruiting body comprising at least 5 %, preferably at least 7 %, more preferably at least 10 %, even more preferably at least 12 % of protein per gram of wet sample/fresh weight; and/or
comprising above 21 %, more preferably above 25 % of protein per gram of dry weight.

12. The method according to any one of claims 1 to 9, or the fruiting body according to claim 10 or 11, wherein the fungus is from the species *Laetiporus sulphureus.*

13. A substrate comprising:
a) a cellulose component;
b) an amino acid component; and
c) a mineral component;
d) and, optionally, a fibrous component.

14. The substrate according to claim 13, further comprising a predetermined water content.

15. The substrate according to claim 14, wherein the predetermined water content is between 30 % to 70 %, preferably between 35 % to 55 %, more preferably 40 % to 50 %, even more preferably 40 % to 45 %.
